# EUROPEAN PATENT SPECIFICATION

(11) **EP 1 469 721 B1**
(45) Date of publication and mention of the grant of the patent: **06.04.2011**
(21) Application number: 01986449.5
(22) Date of filing: 26.09.2001
(51) Int. Cl.: A01K 67/027

(54) **METHOD OF DETERMINING THE IMMUNOGENICITY OF PROTEINS PRODUCING AN ALTERED IMMUNOGENIC RESPONSE**
METHODE ZUR BESTIMMUNG DER IMMUNOGENITÄT VON PROTEINEN, DIE EINE VERÄNDERTE IMMUNANTWORT HERVORRUFEN
METHODE DE DETERMINATION DE L'IMMUNOGENICITE DE PROTEINES PRODUISANT UNE REPONSE IMMUNOGENE MODIFIEE

(30) Priority: 02.10.2000 US 677822; 23.01.2001 US 768080
(43) Date of publication of application: 27.10.2004
(73) Proprietor: Danisco US Inc., Palo Alto, CA 94304 (US)
(72) Inventor: ESTELL, David, A., San Mateo, CA 94403 (US); HARDING, Fiona, A., Santa Clara, CA 95050 (US)
(74) Representative: Kiddle, Simon John
(86) International application number: PCT/US2001/030062
(87) International publication number: WO 2002/040997

(56) References cited:
- WO-A-00/34317
- WO-A-92/10755
- WO-A-99/53078
- CHEN DAN ET AL: "Characterization of HLA DR3/DQ2 transgenic mice: A potential humanized animal model for autoimmune disease studies." EUROPEAN JOURNAL OF IMMUNOLOGY, vol. 33, no. 1, January 2003 (2003-01), pages 172-182, XP002258227 ISSN: 0014-2980 (ISSN print)
- GRUSBY MICHAEL J ET AL: "Mice lacking major histocompatibility complex class I and class II molecules" PROCEEDINGS OF THE NATIONAL ACADEMY OF SCIENCES OF THE UNITED STATES, vol. 90, no. 9, 1993, pages 3913-3917, XP002258228 1993 ISSN: 0027-8424
- SONDERSTRUP G ET AL: "HLA Class II Tansgenic Mice: Models of the Human CD4+ T-cell immune response" IMMUNOLOGICAL REVIEWS, vol. 172, 1999, pages 335-343, XP001155674

## Description

### BACKGROUND OF THE INVENTION

Proteins used in industrial, pharmaceutical and commercial applications are of increasing prevalence. As a result, the increased exposure due to this prevalence has been responsible for some safety hazards caused by the sensitization of certain persons to those peptides, whereupon subsequent exposure causes extreme allergic reactions which can be injurious and even fatal. For example, proteases are known to cause dangerous hypersensitivity in some individuals. As a result, despite the usefulness of proteases in industry, e.g., in laundry detergents, cosmetics, textile treatment etc., and the extensive research performed in the field to provide improved proteases which have, for example, more effective stain removal under detergency conditions; the use of proteases in industry has been problematic due to their ability to produce a hypersensitive allergenic response in some humans.

Much work has been done to alleviate these problems. Among the strategies explored to reduce immunogenic potential of protease use have been improved production processes which reduce potential contact by controlling and minimizing workplace concentrations of dust particles or aerosol carrying airborne protease, improved granulation processes which reduce the amount of dust or aerosol actually produced from the protease product, and improved recovery processes to reduce the level of potentially allergenic contaminants in the final product. However, efforts to reduce the allergenicity of protease, per se, have been relatively unsuccessful. Alternatively, efforts have been made to mask epitopes in protease which are recognized by immunoglobulin E (IgE) in hypersensitive individuals (PCT Publication No. WO 92/10755) or to enlarge or change the nature of the antigenic determinants by attaching polymers or peptides/proteins to the problematic protease.

When an adaptive immune response occurs in an exaggerated or inappropriate form, the individual experiencing the reaction is said to be hypersensitive. Hypersensitivity reactions are the result of normally beneficial immune responses acting inappropriately and sometimes cause inflammatory reactions and tissue damage. They can be provoked by many antigens; and the cause of a hypersensitivity reaction will vary from one individual to the next. Hypersensitivity does not normally manifest itself upon first contact with the antigen, but usually appears upon subsequent contact. One form of hypersensitivity occurs when an IgE response is directed against innocuous environmental antigens, such as pollen, dust-mites or animal dander. The resulting release of pharmacological mediators by IgE-sensitized mast cells produces an acute inflammatory reaction with symptoms such as asthma or rhinitis.

Nonetheless, a strategy comprising modifying the IgE sites will not generally be successful in preventing the cause of the initial sensitization reaction. Accordingly, such strategies, while perhaps neutralizing or reducing the severity of the subsequent hypersensitivity reaction, will not reduce the number or persons actually sensitized. For example, when a person is known to be hypersensitive to a certain antigen, the general, and only safe, manner of dealing with such a situation is to isolate the hypersensitive person from the antigen as completely as possible. Indeed, any other course of action would be dangerous to the health of the hypersensitive individual. Thus, while reducing the danger of a specific protein for a hypersensitive individual is important, for industrial purposes it would be far more valuable to render a protein incapable of initiating the hypersensitivity reaction in the first place.

T-lymphocytes (T-cells) are key players in the induction and regulation of immune responses and in the execution of immunological effector functions. Specific immunity against infectious agents and tumors is known to be dependent on these cells and they are believed to contribute to the healing of injuries. On the other hand, failure to control these responses can lead to auto aggression. In general, antigen is presented to T-cells in the form of antigen presenting cells which, through a variety of cell surface mechanisms, capture and display antigen or partial antigen in a manner suitable for antigen recognition by the T-cell. Upon recognition of a specific epitope by the receptors on the surface of the T-cells (T-cell receptors), the T-cells begin a series of complex interactions, including proliferation, which result in the production of antibody by B-cells. While T-cells and B-cells are both activated by antigenic epitopes which exist on a given protein or peptide, the actual epitopes recognized by these mononuclear cells are generally not identical, In fact, the epitope which activates a T-cell to initiate the creation of immunologic diversity is quite often not the same epitope which is later recognized by B-cells in the course of the immunologic response. Thus, with respect to hypersensitivity, while the specific antigenic interaction between the T-cell and the antigen is a critical element in the initiation of the immune response to antigenic exposure, the specifics of that interaction, i.e., the epitope recognized, is often not relevant to subsequent development of a full blown allergic reaction.

PCT Publication No. WO 96/40791 discloses a process for producing polyalkylene oxide-polypeptide conjugates with reduced allergenicity using polyalkylene oxide as a starting material.

PCT Publication No. WO 97/30148 discloses a polypeptide conjugate with reduced allergenicity which comprises one polymeric carrier molecule having two or more polypeptide molecules coupled covalently thereto.

PCT Publication No. WO 96/17929 discloses a process for producing polypeptides with reduced allergenicity comprising the step of conjugating from 1 to 30 polymolecules to a parent polypeptide.

PCT Publication No. WO 92/10755 discloses a method of producing protein variants evoking a reduced immunogenic response in animals. In this application, the proteins of interest, a series of proteases and variants thereof, were used to immunize rats. The sera from the rats was then used to measure the reactivity of the polyclonal antibodies already produced and present in the immunized sera to the protein of interest and variants thereof. From these results, it was possible to determine whether the antibodies in the preparation were comparatively more or less reactive with the protein and its variants, thus permitting an analysis of which changes in the protein are likely to neutralize or reduce the ability of the lg to bind. From these tests on rats, the conclusion was arrived at that changing any of subtilisin 309 residues corresponding to 127, 128, 129, 130, 131, 151,138, 151,152,153, 154,161, 162,183,167,168, 169,170, 171, 172, 173,174, 175, 176,186, 193, 94,195,196,197, 247, 251, 261 will result In a change in the immunological potential.

PCT Publication No. WO 94/10191 discloses low allergenic proteins comprising oligomeric forms of the parent monomeric protein, wherein the oligomer has substantially retained its activity,

WO 00/34317 discloses methods of rendering therapeutic proteins non-or less immunogenic by removing previously identified T cell epitopes by e.g. amino acid modification. It also discloses modified variants of the thus treated proteins. Testing the activity of modified variant therapeutic proteins may be made using transgenic mice reconstituted with (parts of) the immune system from the species chosen to receive therapeutic proteins.

While some studies have provided methods of reducing the allergenicity of certain proteins and identification of epitopes which cause allergic reactions In some individuals, the assays used to identify these epitopes generally involve measurement of IgE and IgG antibody in blood sera previously exposed to the antigen. However, once an lg reaction has been initiated, sensitization has already occurred. Accordingly, there is a need for a method of determining epitopes which cause sensitization in the first place, as neutralization of these epitopes will result in significantly less possibility for sensitization to occur, thus reducing the possibility of initial sensitization. There is also a need to produce proteins which produce an enhanced immunogenic response, and a need to identify naturally occurring proteins which produce a low immunogenic response. This Invention meets these and other needs.

### SUMMARY OF THE INVENTION

The invention provides a method to determine the allergenic potential of an engineered protein, the method comprising the steps of,
a) immunizing a first transgenic mouse with a protein of interest and immunizing a second transgenic mouse with an engineered protein wherein said engineered protein is a variant of said protein of interest and said protein of interest includes a T-cell epitope wherein the variant differs from the protein of interest by having an altered T cell epitope;
b) collecting serum of said first and said second immunized transgenic mice;
c) measuring the serum for antigen specific immunoglobulins; and
d) comparing the immunogenic response of said variant and said protein of interest;
wherein the first transgenic mouse and second transgenic mouse are HLA DR3/DQ2, and wherein the variant and the protein of interest produce a different immunogenic response in said transgenic mice.

The antigen specific immunoglobulin may be IgG.

In certain embodiments, the HLA DR3/DQ2 transgenic mice have been backcrossed with mice lacking the expression of endogenous I-A class II molecules.

The protein or polypeptide of Interest can be any protein or polypeptide of interest. In one aspect, the polypeptide is selected from the group consisting of enzymes, hormones, factors, vaccines and cytokines. As indicated herein, the polypeptide of interest may be an enzyme. In one embodiment, the enzyme is selected from the group consisting of lipase, cellulase, endo-glucosidase H, protease, carbohydrase, reductase, oxidase, isomerase, transferase, kinase and phosphatase. In preferred embodiments, the polypeptide of interest and the variant of said polypeptide of interest each comprise at least some of the same activity. For example, if a variant of a protease is provided, said variant will produce an altered immunogenic response, but will retain detectable, and preferably comparable, protease activity.

The T-cell epitope may be altered in a number of ways including by amino acid substitutions, deletions, additions and combinations thereof. Preferably, the T-cell epitope is altered by having amino acid substitutions. In one embodiment herein, the amino acid substitutions are made to corresponding amino acids of a homolog of the polypeptide of interest, wherein the homolog does not comprise the same T-cell epitope in the corresponding position as the polypeptide of interest In one aspect, the terminal portion of the polypeptide of interest comprising at least one T-cell epitope is replaced with a corresponding terminal portion of the homolog of the polypeptide of interest, wherein the replacement produces said different immunogenic response.

The method of the invention may be used to alter, the immunogenicity of a polypeptide of interest by determining the immunogenicity of said polypeptide; identifying a T-cell epitope in a said polypeptide; and altering said T-cell epitope so as to alter the immunogencity of said polypeptide. As described herein, said altering can be done by altering a single amino acid or switching a portion of the polypeptide of interest with a corresponding portion of a homolog, wherein the switch produces an altered immunogenic response.

Other aspects of the invention will be understood by the skilled artisan by the following description.

### BRIEF DESCRIPTION OF THE DRAWINGS

Figs. 1 A, B1, B2 and B3 illustrate the DNA (SEQ ID:NO 1) and amino acid (SEQ ID: NO 2) sequence for *Bacillus amyloliquefaciens subtilisin* (BPN') and a partial restriction map of this gene.
Fig. 2 illustrates the conserved amino acid residues among subtilisins from *Bacillus amyloliquefaciens* (SEQ ID:NO 3) and *Bacillus lentus* (wild-type) (SEQ ID:NO 4).
Figs. 3A and 3B illustrate an amino acid sequence alignment of subtilisin type proteases from *Bacillus amyloliquefaciens* (BPN'), *Bacillus subtilis, Bacillus licheniformis* (SEQ ID:NO 5) and *Bacillus lentus.* The symbol * denotes the absence of specific amino acid residues as compared to subtilisin BPN'.
Fig. 4 illustrates the additive T-cell response of 16 peripheral mononuclear blood samples to peptides corresponding to the *Bacillus lentus* protease (GG36). Peptide E05 includes the region comprising residues corresponding to 170-173 in protease from *Bacillus amyloliquefaciens.*
Fig. 5 illustrates the additive T-cell response of 10 peripheral mononuclear blood samples to peptides corresponding to the human subtilisin molecule. Peptides F10, F9, F8 and F7 all contain the amino acid sequence DQMD corresponding to the region comprising residues corresponding to 170-173 in protease from *Bacillus amyloliquefaciens* in the sequence alignment of Fig. 3.
Fig. 6A and 6B/6C illustrate amino acid strings corresponding to peptides derived from the sequence of *Bacillus lentus* protease and a human subtilisin, respectively.
Fig. 7 illustrates the amino acid sequence of human subtilisin (SEQ ID:NO 6).
Fig. 8 illustrates an amino acid sequence alignment of BPN' (*Bacillus amyloliquefaciens*) protease, SAVINASE *(Bacillus lentus)* protease and human subtilisin (S2HSBT).
Fig. 9 illustrates the T-cell response to peptides derived from *Bacillus lentus* protease in a sample taken from an individual known to be hypersensitive to *Bacillus lentus* protease. Peptide E05 represents the region corresponding to 170-173 in protease from *Bacillus amyloliquefaciens.*
Fig. 10 illustrates the T-cell response to various alanine substitutions in the E05 *Bacillus lentus* protease peptide set in a sample taken from an individual known to be hypersensitive to *Bacillus lentus* protease.
Fig. 11 illustrates the T-cell response to various alanine substitutions in the E05 protease peptide (an embodiment of the T-cell epitope designated unmodified sequence) set in a sample taken from an individual known to be hypersensitive to the protease; the sequences for each peptide are also shown.

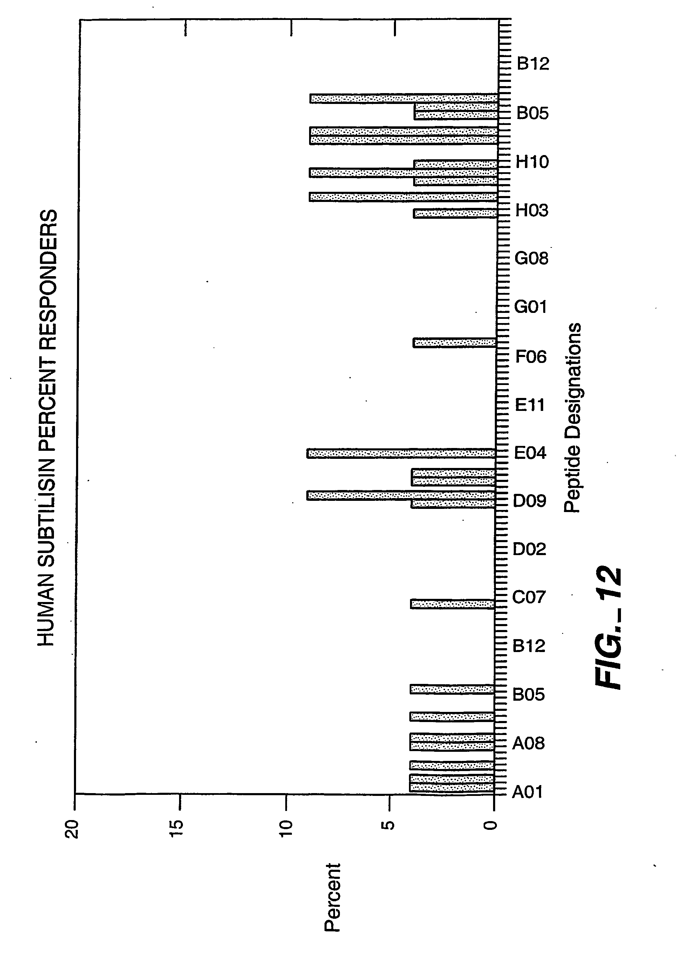

Fig. 12 illustrates the percent responders to the human subtilisin molecule.
Fig. 13A illustrates the T-cell response of peptides derived from *Humicola insolens* endogluconase (Accession number A23635). Peptides A02 and F06 represent the region corresponding to residues 70-84 and 37-51, respectively, embodiments of the T-cell epitope, of *Humicola insolens* endogluconase, wherein the full length sequence is shown In Fig.13B and A02 and F06 are shown underlined and in bold.
Fig. 14A illustrates the T-cell response to peptides derived from *Thermomyces lanuginosa* lipase (Accession number AAC08588 and PID number g2997733). Peptides B02 and C06 represent the regions corresponding to residues 83-100 and 108-121, respectively, embodiments of the T-cell epitope, of *Thermomyces lanuginosa* lipase, wherein the full length sequence is shown in Fig.14B and B02 and C06 are shown underlined and in bold.
Fig. 15A illustrates the T-cell response to peptides derived from *Streptomyces plicatus* endo-beta-N-acetylglucosaminidase. (Accession number P04067). Peptide C06 represents the region corresponding to residues 126-140, an embodiment of the T-cell epitope, of *Streptomyces plicatus* endo-beta-N-acetylglucosaminidase, wherein the full length sequence is shown in Fig. 15B and C06 is shown underlined and in bold.
Fig. 16 illustrates the T-cell response to peptides derived from BPN' compiled for 22 individuals, wherein the sequences of preferred T-cell epitopes are indicated.
Fig. 17 illustrates the T-cell response to peptides derived from GG36 complied for 22 individuals, wherein the sequences of embodiments of T-cell epitopes are indicated, GSISYPARYANAMAVGA and GAGLDIVAPGVNVQS being preferred.
Fig. 18 is an embodiment of a hybrid protein provided herein, where the N-terminus comprises N-terminal GG36 sequence and the C-terminus comprises C-terminal BPN' sequence, and wherein a comparison of the sequences with those shown in Fig. 8 indicates that the hybrid formed omits preferred T-cell epitopes of each protein.
Figure 19 is a comparison of ELISA titers for *B*. *amyloliquefaciens* subtilisin and the same subtilisin but engineered to contain a T-cell epitope from *B*. lentis subtilisin. Figure 19a represents the titer at 4 weeks; Figure 19b at 6 weeks, Figure 19c at 8 weeks and Figure 19d at 10 weeks.
Figure 20 is a time course study of ELISA titers for *B*. *amylo*/*iquefaciens* subtilisin and the same subtilisin but engineered to contain a T-cell epitope from *B. lentis* subtilisin. Figure 20a represents the titer for a 1µg dose of enzyme, Figure 20b a 5 µg dose and Figure 20c a 20 µg dose.

### DETAILED DESCRIPTION OF THE INVENTION

According to the present invention, a method for identifying T-cell epitopes is provided. Moreover, proteins including naturally occurring proteins which have relatively impotent or potent T-cell epitopes or no T-cell epitopes may be identified in accordance with the methods of the present invention. Thus, the present invention allows the identification and production of proteins which produce immunogenic responses as desired, including naturally occurring proteins as well as proteins which have been mutated to produce the appropriate response. It is understood that the terms protein, polypeptide and peptide are sometimes used herein interchangeably. Wherein a peptide is a portion of protein, the skilled artisan can understand this by the context in which the term is used.

"Antigen presenting cell" as used herein means a cell of the immune system which present antigen on their surface which is recognizable by receptors on the surface of T-cells. Examples of antigen presenting cells are dendritic cells, interdigitating cells, activated B-cells and macrophages.

"T-cell proliferation" as used herein means the number of T-cells produced during the incubation of T-cells with the antigen presenting cells, with or without antigen.

"Baseline T-cell proliferation" as used herein means T-cell proliferation which is normally seen In an individual in response to exposure to antigen presenting cells in the absence of peptide or protein antigen. For the purposes herein, the baseline T-cell proliferation level was determined on a per sample basis for each individual as the proliferation of T-cells In response to antigen presenting cells in the absence of antigens.

"T-cell epitope" means a feature of a peptide or protein which Is recognized by a T-cell receptor in the initiation of an immunologic response to the peptide comprising that antigen. Recognition of a T-cell epitope by a T-cell is generally believed to be via a mechanism wherein T-cells recognize peptide fragments of antigens which are bound to class I or class II major histocompatability (MHC) molecules expressed on antigen-presenting cells (*see e.g*., Moeller, G. ed., "Antigenic Requirements for Activation of MHC-Restricted Responses," immunological Review, Vol. 98, p.187 (Copenhagen; Munksgaard) (1987).

"Sample" as used herein comprises mononuclear cells which are naïve, *i.e*., not sensitized, to the antigen in question.

"Homolog" as used herein means a protein or enzyme which has similar catalytic action, structure and/or use as the protein of interest. For purposes of this invention, a homolog and a protein of interest are not necessarily related evolutionarily, *e.g*., same functional protein from different species. It is desirable to find a homolog that has a tertiary and/or primary structure similar to the protein of Interest as replacement of the epitope in the protein of Interest with an analogous segment from the homolog will reduce the disruptiveness of the change. Thus, closely homologous enzymes will provide the most desirable source of epitope substitutions. Alternatively, If possible, it is advantageous to look to human analogs for a given protein. For example, substituting a specific epitope in a bacterial subtilisin with a sequence from a human analog to subtilisin (*i.e*., human subtilisin) should result in less allergenicity in the bacterial protein.

An "analogous" sequence may be determined by ensuring that the replacement amino acids show a similar function, the tertiary structure and/or conserved residues to the amino acids in the protein of interest at or near the epitope. Thus, where the epitope region contains, for example, an alpha-helix or a beta-sheet structure, the replacement amino acids should maintain that specific structure.

The epitopes determined according to the assay provided herein are then modified to reduce or augment the immunologic potential of the protein of interest

Preferably, the epitope is modified In one of the following ways: (a) the amino acid sequence of the epitope is substituted with an analogous sequence from a human homolog to the protein of interest; (b) the amino acid sequence of the epitope is substituted with an analogous sequence from a non-human homolog to the protein of interest, which analogous sequence produces a lesser immunogenic, e.g., allergenic, response due to T-cell epitope recognition than that of the protein of interest; (c) the amino acid sequence of the epitope Is substituted with a sequence which substantially mimics the major tertiary structure attributes of the epitope, but which produces a lesser immunogenic, e.g., allergenic, response due to T-cell epitope recognition than that of the protein of interest; or (d) with any sequence which produces lesser immunogenic, e.g., allergenic, response due to T-cell epitope recognition than that of the protein of interest.

However, one of skill will readily recognize that epitopes can be modified in other ways depending on the desired outcome. For example, if a T-cell vaccine is desired, it is contemplated the amino acid sequence of an epitope will be substituted with amino acids which increase the immulogic response to the peptide via enhanced MHC binding and/or T-cell recognition. In another example, if altering an autoimmune response against self antigens is desired, it is contemplated the amino acid sequence of an epitope will be substituted with amino acids that decrease or cause a shift in an inflammatory or other immune response.

The method of the present invention may be applied, to all proteins against which it is desired to modulate the immunogenic response, for example, peptides to be used as T-cell vaccines, or peptides or proteins to be used as therapeutic agents against, e.g., cancer, infectious diseases and autoimmune diseases. One of skill in the art will readily recognize the proteins and peptides of this invention are not necessarily native proteins and peptides. Indeed, in one embodiment of this invention, the assay described herein is used to determine the immunologic response of proteins from shuffled genes. For descriptions of gene shuffling and expression of such genes see, Stemmer, Proc. Nat'l Acad. Sci. USA 91:10747 (1994); Patten, et al., Current Opinion in Biotechnol. 8:724 (1997); Kuchner & Arnold, Trends Biotechnol. 15:523 (1997); Moore, et al., J. Mol, Biol. 272:336 (1997); zhao, at al., Nature Biotechnol. 16:258 (1998); Giver, et al., Proc. Nat'l Acad. Sci. USA 95:12809 (1998); Harayama, Trends Biotechnol. 16:76 (1998); Lin, et a/., Biotechnol., Prog.15:467 (1999); and Sun, J. Comput Biol 6:77 (1999). The assay is used to predict the immunologic response of proteins encoded by shuffled genes. Once determined, the protein can be altered to modulate the immunoigic response to that protein.

In addition to the above proteins and peptides, the present invention can be used to reduce the allergenicity of proteins. These proteins include, but are not limited to, glucanases, lipases, cellulases, endo-glucosidase Hs (endo-H), proteases, carbohydrases, reductases, oxidases, isomerases, transferases, kinases, phosphatases, amylases, etc. In addition to reducing the allergenicity to an animal, such as a human, of naturally occurring amino acid sequences, this invention encompasses reducing the allergenicity of a mutated human protein, e.g., a protein that has been altered to change the functional activity of the protein. In many instances, the mutation of human proteins to e.g., increase activity, results in the incorporation of new T-cell epitope In the mutated protein. The assay of this Invention can be used to determine the presence of the new T-cell epitope and determine substitute amino acids that will reduce the allergenicity of the mutated protein.

Although the method of this invention may be applied to the above proteins and many others, for the sake of simplicity, the following will describe a particularly preferred embodiment of the invention, the modification of protease. Proteases are carbonyl hydrolases which generally act to cleave peptide bonds of proteins or peptides. As used herein, "protease means a naturally-occurring protease or a recombinant protease. Naturally-occurring proteases include a-aminoacylpeptide hydrolase, peptidylamino acid hydrolase, acylamino hydrolase, serine carboxypeptidase, metallocarboxypeptidase, thiol proteinase, carboxylproteinase and metalloproteinase. Serine, metallo, thiol and acid proteases are included, as well as endo and exo-proteases.

In one embodiment herein, hybrid polypeptides are provided. "Hybrid polypeptides" are proteins engineered from at least two different proteins, which are preferably homologs of one another. For example, a preferred hybrid polypeptide might have the N-terminus of a protein and the C-terminus of a homolog of the protein. In a preferred embodiment, the two terminal ends can be combined to correspond to the full-length active protein. In a preferred embodiment, the homologs share substantial similarity but do not have identical T-cell epitopes. Therefore, in one embodiment, for example, a polypeptide of interest having one or more T-cell epitopes in the C-terminus may have the C-terminus replaced with the C-terminus of a homolog having a less potent T-cell epitope in the C-terminus, less T-cell epitopes, or no T-cell epitope in the C-terminus. Thus, the skilled artisan understands that by being able to identify T-cell epitopes among homologs, a variety of variants producing different immunogenic responses can be formed. Moreover, it is understood that internal portions, and more than one homolog can be used to produce the variants of the present invention.

More generally, the variants provided herein can be derived from the precursor amino acid sequence by the substitution, deletion, insertion, or combination thereof of one or more amino acids of the precursor amino acid sequence. Such modification is preferably of the "precursor DNA sequence" which encodes the amino acid sequence of the precursor enzyme, but can be by the manipulation of the precursor protein. Suitable methods for such manipulation of the precursor DNA sequence include methods disclosed herein, as well as methods known to those skilled in the art (see, for example, EP 0 328299, WO89/06279 and the US patents and applications already referenced herein).

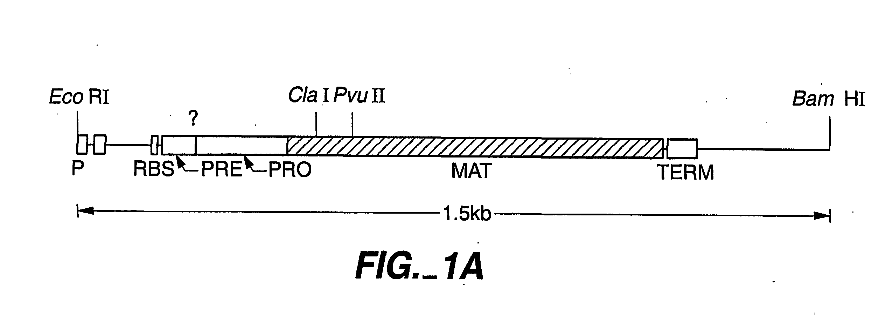

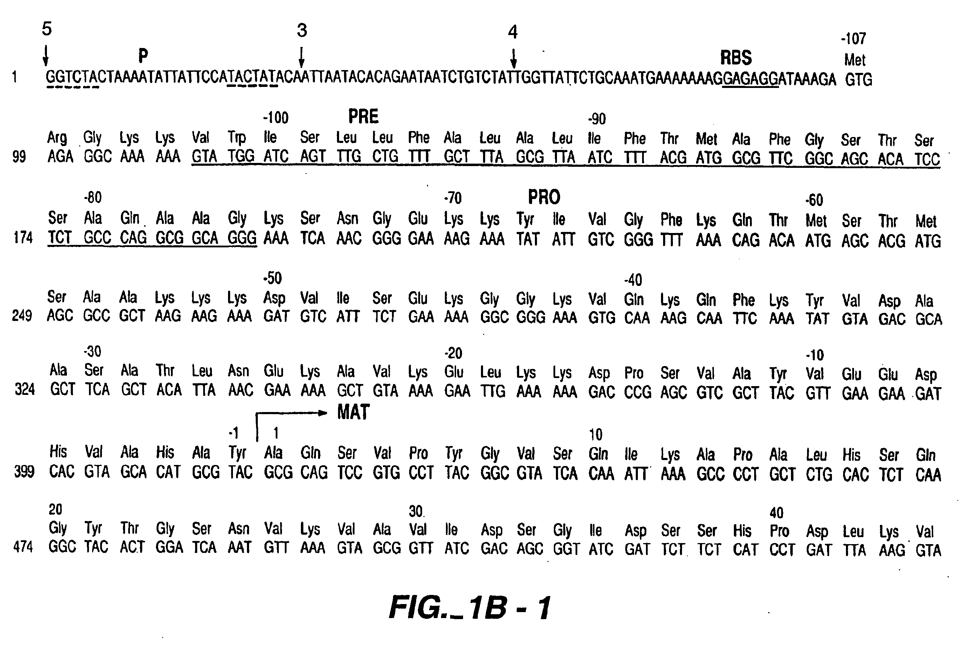

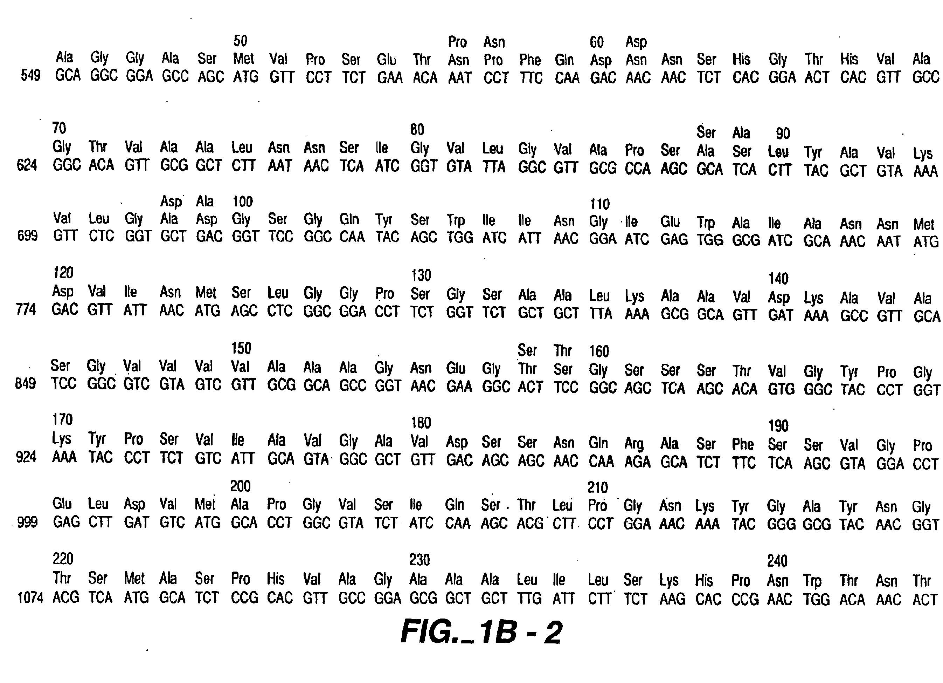

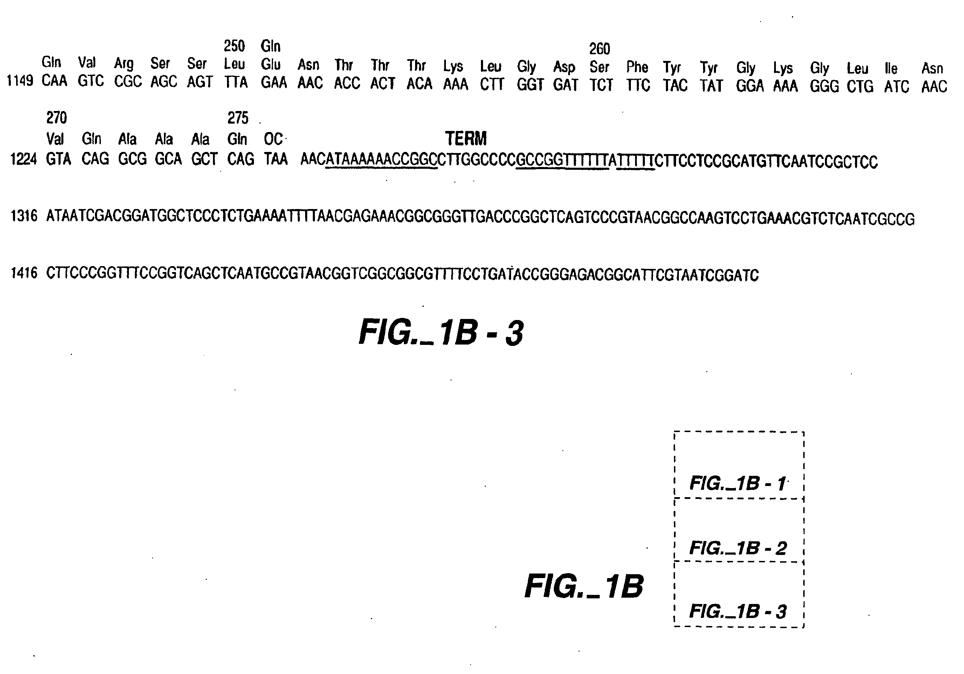

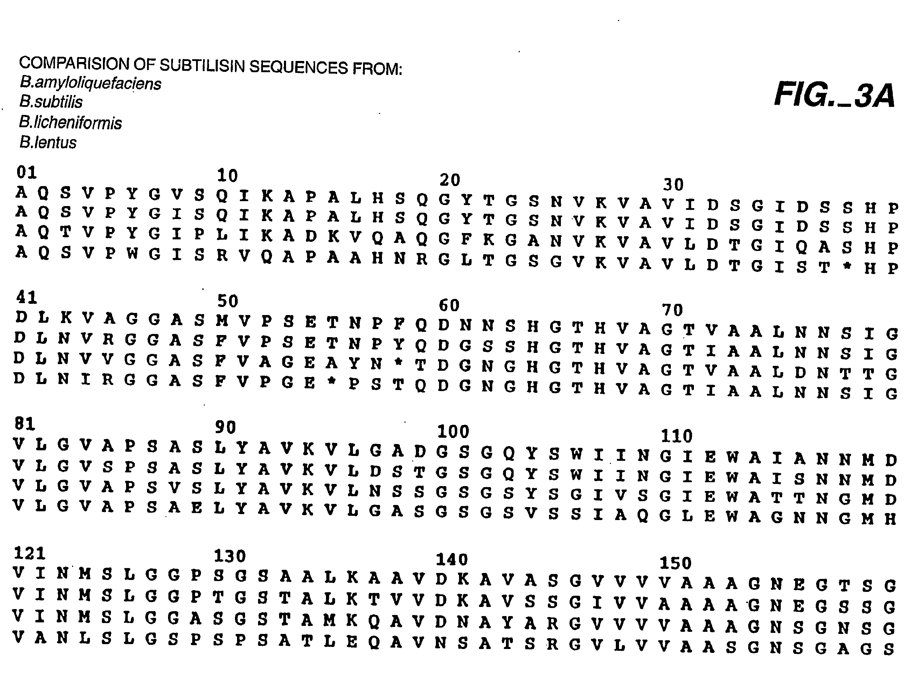

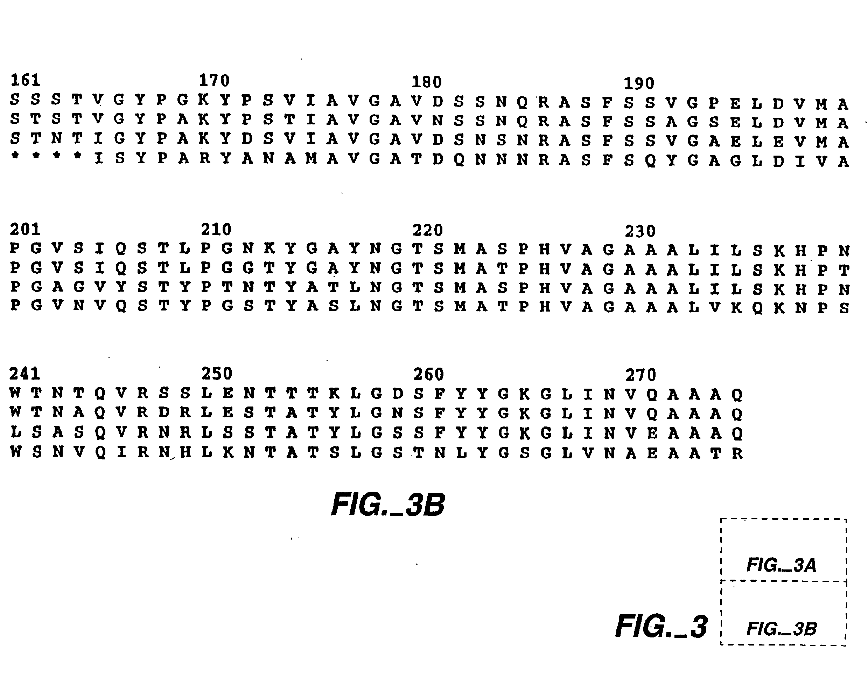

Subtilisins are bacterial or fungal proteases which generally act to cleave peptide bonds of protein or peptides. As used herein, "subtilisin" means a naturally-occurring subtilisin or a recombinant subtilisin. A series of naturally-occurring subtilisins is known to be produced and often secreted by various microbial species. Amino acid sequences of the members of this series are not entirely homologous. However, the subtilisins in this series exhibit the same or similar type of proteolytic activity. This class of serine proteases shares a common amino acid sequence defining a catalytic triad which distinguishes them from the chymotrypsin related class of serine proteases. The subtilisins and chymotrypsin related serine proteases both have a catalytic triad comprising aspartate, histidine and serine. In the subtilisin related proteases the relative order of these amino acids, reading from the amino to carboxy terminus, is aspartate-histidine-serine. In the chymotrypsin related proteases, the relative order, however, is histidine-aspartate-serine. Thus, subtilisin herein refers to a serine protease having the catalytic triad of subtilisin related proteases. Examples include but are not limited to the subtilisins identified in Fig. 3 herein. Generally and for purposes of the present invention, numbering of the amino acids in proteases corresponds to the numbers assigned to the mature *Bacillus amyloliquefaciens* subtilisin sequence presented in Fig. 1.

"Recombinant", "recombinant subtilisin" or "recombinant protease" refer to a subtilisin or protease in which the DNA sequence encoding the subtilisin or protease is modified to produce a variant (or mutant) DNA sequence which encodes the substitution, deletion or insertion of one or more amino acids in the naturally-occurring amino acid sequence. Suitable methods to produce such modification, and which may be combined with those disclosed herein, include those disclosed in US Patent 4,760,025 (RE 34,606), US Patent 5,204,015 and US Patent 5,185,258.

"Non-human subtilisins" and the DNA encoding them may be obtained from many procaryotic and eucaryotic organisms. Suitable examples of procaryotic organisms include gram negative organisms such as *E. coli* or *Pseudomonas* and gram positive bacteria such as *Micrococcus* or *Bacillus*. Examples of eucaryotic organisms from which subtilisin and their genes may be obtained include yeast such as *Saccharomyces cerevisiae,* fungi such as *Aspergillus* sp.

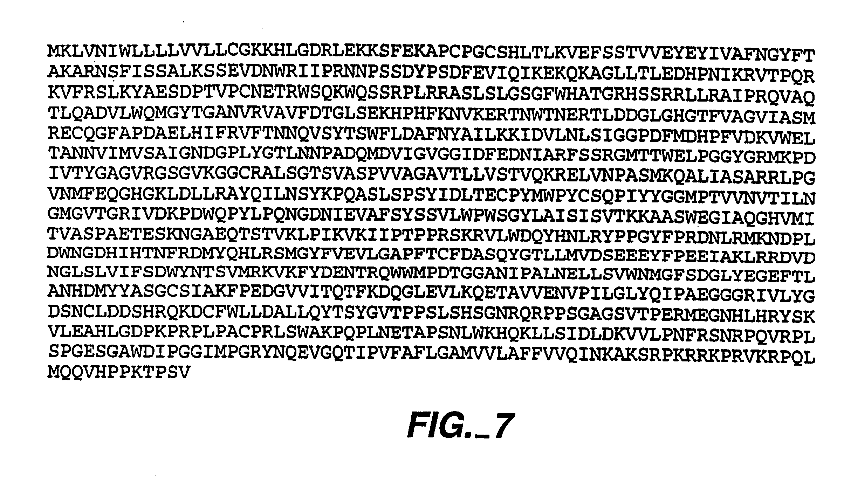

"Human subtilisin" means proteins of human origin which have subtilisin type catalytic activity, e.g., the kexin family of human derived proteases. An example of such a protein is represented by the sequence in Fig. 7. Additionally, derivatives or homologs of proteins provided herein, including those from non-human sources such as mouse or rabbit, which retain the essential activity of the peptide, such as the ability to hydrolyze peptide bonds, etc., have at least 50%, preferably at least 65% and most preferably at least 80%, more preferably at least 90%, and sometimes as much as 95 or 98% homology to the polypeptide of interest. In one embodiment, the polypeptide of interest is shown in the Figures.

The amino acid position numbers used herein refer to those assigned to the mature *Bacillus amyloliquefaciens* subtilisin sequence presented in Fig. 1. The invention, however, is not limited to the mutation of this particular subtilisin but extends to precursor proteases containing amino acid residues at positions which are "equivalent" to the particular identified residues in *Bacillus amyloliquefaciens* subtilisin. In a preferred embodiment of the present invention, the precursor protease is *Bacillus lentus* subtilisin and the substitutions, deletions or insertions are made at the equivalent amino acid residue in *B. lentus* corresponding to those listed above.

A residue (amino acid) of a precursor protease is equivalent to a residue of *Bacillus amyloliquefaciens* subtilisin if it is either homologous (i.e., corresponding in position in either primary or tertiary structure) or analogous to a specific residue or portion of that residue in *Bacillus amyloliquefaciens* subtilisin (i.e., having the same or similar functional capacity to combine, react, or interact chemically). "Corresponding" as used herein generally refers to an analogous position along the peptide.

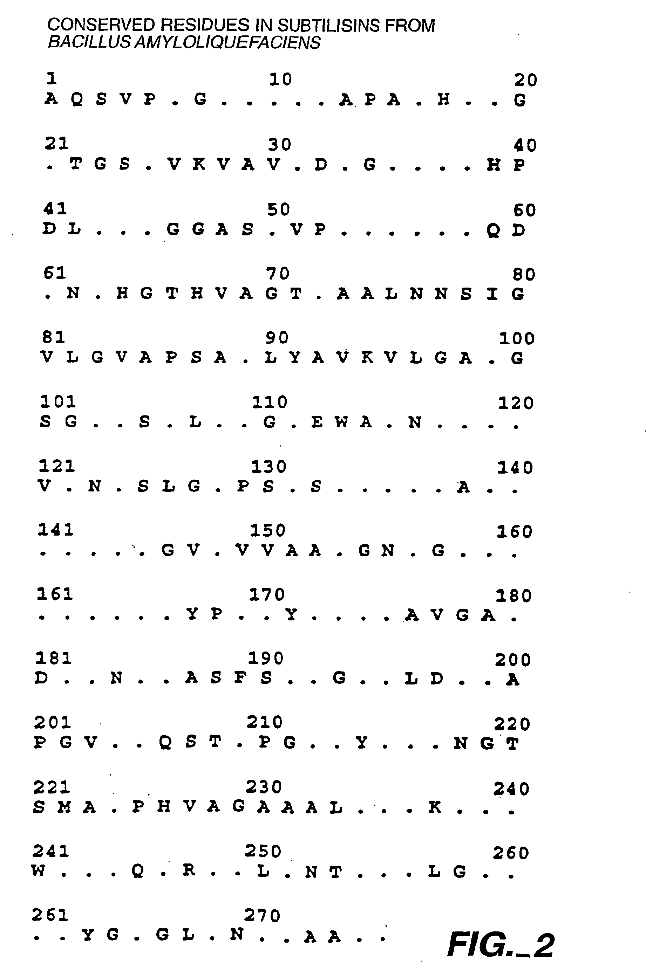

In order to establish homology to primary structure, the amino acid sequence of a precursor protease is directly compared to the *Bacillus amyloliquefaciens* subtilisin primary sequence and particularly to a set of residues known to be invariant in subtilisins for which the sequence is known. For example, Fig. 2 herein shows the conserved residues as between *B*. *amyloliquefaciens* subtilisin and *B. lentus* subtilisin. After aligning the conserved residues, allowing for necessary insertions and deletions in order to maintain alignment (i.e., avoiding the elimination of conserved residues through arbitrary deletion and insertion), the residues equivalent to particular amino acids in the primary sequence of *Bacillus amyloliquefaciens* subtilisin are defined. Alignment of conserved residues preferably should conserve 100% of such residues. However, alignment of greater than 75% or as little as 50% of conserved residues is also adequate to define equivalent residues. Conservation of the catalytic triad, Asp32/His64/Ser221 should be maintained.

For example, the amino acid sequence of subtilisin from *Bacillus amyloliquefaciens, Bacillus subtilis, Bacillus licheniformis (carlsbergensis)* and *Bacillus lentus* can be aligned to provide the maximum amount of homology between amino acid sequences. A comparison of these sequences shows that there are a number of conserved residues contained in each sequence. The conserved residues as between BPN' and *B. lentus* are identified in Fig. 2.

These conserved residues, thus, may be used to define the corresponding equivalent amino acid residues of *Bacillus amyloliquefaciens* subtilisin in other subtilisins such as subtilisin from *Bacillus lentus* (PCT Publication No. W089/06279 published July 13, 1989), the preferred protease precursor enzyme herein, or the subtilisin referred to as PB92 (EP 0 328 299), which is highly homologous to the preferred *Bacillus lentus* subtilisin. The amino acid sequences of certain of these subtilisins are aligned in Figs. 3A and 3B with the sequence of *Bacillus amyloliquefaciens* subtilisin to produce the maximum homology of conserved residues. As can be seen, there are a number of deletions in the sequence of *Bacillus lentus* as compared to *Bacillus amyloliquefaciens* subtilisin. Thus, for example, the equivalent amino acid for Val165 in *Bacillus amyloliquefaciens* subtilisin in the other subtilisins is isoleucine for *B. lentus* and *B*. *licheniformis.*

Thus, for example, the amino acid at position +170 is lysine (K) in both *B. amyloliquefaciens* and *B. licheniformis* subtilisins and arginine (R) in Savinase. In one embodiment of the protease variants of the invention, however, the amino acid equivalent to +170 in *Bacillus amyloliquefaciens* subtilisin is substituted with aspartic acid (D). The abbreviations and one letter codes for all amino acids in the present invention conform to the Patentin User Manual (GenBank, Mountain View, CA) 1990, p.101.

Homologous sequences can also be determined by using a "sequence comparison algorithm." Optimal alignment of sequences for comparison can be conducted, *e.g*., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, Wl), or by visual inspection.

An example of an algorithm that is suitable for determining sequence similarity is the BLAST algorithm, which is described in Altschul, et al., J. Mol. Biol. 215:403-410 (1990). Software for performing BLAST analyses is publicly available through the National Center for Biotechnology Information (httpJ//www.ncbi.nlm.nih.gov/). This algorithm involves first identifying high scoring sequence pairs (HSPs) by identifying short words of length W in the query sequence that either match or satisfy some positive-valued threshold score T when aligned with a word of the same length in a database sequence. These initial neighborhood word hits act as starting points to find longer HSPs containing them. The word hits are expanded in both directions along each of the two sequences being compared for as far as the cumulative alignment score can be increased. Extension of the word hits is stopped when: the cumulative alignment score falls off by the quantity X from a maximum achieved value; the cumulative score goes to zero or below, or the end of either sequence is reached. The BLAST algorithm parameters W, T, and X determine the sensitivity and speed of the alignment. The BLAST program uses as defaults a wordlength (W) of 11, the BLOSUM62 scoring matrix (see Henikoff & Henikoff, Proc. Natl. Acad. Sci. USA 89:10915 (1989)) alignments (B) of 50, expectation (E) of 10, M'5, N'-4, and a comparison of both strands.

The BLAST algorithm then performs a statistical analysis of the similarity between two sequences (see, e.g., Kariin & Altschul, Proc. Nat'l. Acad. Sci USA 90:5873-5787 (1993)). One measure of similarity provided by the BLAST algorithm is the smallest sum probability (P(N)), which provides an indication of the probability by which a match between two nucleotide or amino acid sequences would occur by chance. For example, an amino acid sequence is considered similar to a protein such as a protease if the smallest sum probability in a comparison of the test amino acid sequence to a protein such as a protease amino acid sequence is less than about 0.1, more preferably less than about 0.01, and most preferably less than about 0.001.

"Equivalent residues" may also be defined by determining homology at the level of tertiary structure for a precursor protein whose tertiary structure has been determined by x-ray crystallography. Equivalent residues are defined as those for which the atomic coordinates of two or more of the main chain atoms of a particular amino acid residue of the precursor protein such as the protease and *Bacillus amyloliquefaciens* subtilisin (N on N, CA on CA, C on C and O on O) are within 0.13nm and preferably 0.1 nm after alignment Alignment is achieved after the best model has been oriented and positioned to give the maximum overlap of atomic coordinates of non-hydrogen protein atoms of the protein such as the protease in question to the *Bacillus, amyloliquefaciens* subtilisin. The best model is the crystallographic model giving the lowest R factor for experimental diffraction data at the highest resolution available.

Equivalent residues which are functionally analogous to a specific residue of *Bacillus amyloliquefaciens* subtilisin are defined as those amino acids of the precursor protein such as a protease which may adopt a conformation such that they either alter, modify or contribute to protein structure, substrate binding or catalysis in a manner defined and attributed to a specific residue of the *Bacillus amyloliquefaciens* subtilisin. Further, they are those residues of the precursor protein, for example, protease (for which a tertiary structure has been obtained by x-ray crystallography) which occupy an analogous position to the extent that, although the main chain atoms of the given residue may not satisfy the criteria of equivalence on the basis of occupying a homologous position, the atomic coordinates of at least two of the side chain atoms of the residue lie with 0.13nm of the corresponding side chain atoms of *Bacillus amyloliquefaciens* subtilisin. The coordinates of the three dimensional structure of *Bacillus amyloliquefaciens* subtilisin are set forth in EPO Publication No. 0 251 446 (equivalent to US Patent 5,182,204) and can be used as outlined above to determine equivalent residues on the level of tertiary structure.

Some of the residues identified for substitution, insertion or deletion are conserved residues whereas others are not In the case of residues which are not conserved, the replacement of one or more amino adds is limited to substitutions which produce a variant which has an amino acid sequence that does not correspond to one found in nature. In the case of conserved residues, such replacements should not result In a naturally-occurring sequence. The variants of the present invention include the mature forms of protein variants, as well as the pro- and prepro- forms of such protein variants. The prepro- forms are the preferred construction since this facilitates the expression, secretion and maturation of the protein variants.

"Prosequence" refers to a sequence of amino acids bound to the N-terminal portion of the mature form of a protein which when removed results in the appearance of the "mature" form of the protein. Many proteolytic enzymes are found in nature as translational proenzyme products and, in the absence of post-translational processing, are expressed in this fashion. A preferred prosequence for producing protein variants such as protease variants is the putative prosequence of *Bacillus amyloliquefaciens* subtilisin, although other prosequences may be used.

A "signal sequence" or "presequence" refers to any sequence of amino acids bound to the N-terminal portion of a protein or to the N-terminal portion of a proprotein which may participate in the secretion of the mature or pro forms of the protein. This definition of signal sequence is a functional one, meant to include all those amino acid sequences encoded by the N-terminal portion of the protein gene which participate in the effectuation of the secretion of protein under native conditions. The present invention utilizes such sequences to effect the secretion of the protein variants as defined herein. One possible signal sequence comprises the first seven amino add residues of the signal sequence from *Bacillus subtilis* subtilisin fused to the remainder of the signal sequence of the subtilisin from *Bacillus lentus* (ATCC 21536).

A "repro" form of a protein variant consists of the mature form of the protein having a prosequence operably linked to the amino terminus of the protein and a "pre" or "signal" sequence operably linked to the amino terminus of the prosequence.

"Expression vector" refers to a DNA construct containing a DNA sequence which is operably linked to a suitable control sequence capable of effecting the expression of said DNA in a suitable host. Such control sequences include a promoter to effect transcription, an optional operator sequence to control such transcription, a sequence encoding suitable mRNA ribosome binding sites and sequences which control termination of transcription and translation. The vector may be a plasmid, a phage particle, or simply a potential genomic insert. Once transformed into a suitable host, the vector may replicate and function independently of the host genome, or may, in some instances, integrate into the genome itself. In the present specification, "plasmid" and "vector' are sometimes used interchangeably as the plasmid is the most commonly used form of vector at present. However, the invention is intended to include such other forms of expression vectors which serve equivalent functions and which are, or become, known in the art

The "host cells" used in the present invention generally are procaryotic or eucaryotic hosts which preferably have been manipulated by the methods disclosed in US Patent 4,760,025 (RE 34,606) to render them incapable of secreting enzymatically active endoprotease. A preferred host cell for expressing protein is the *Bacillus* strain BG2036 which is deficient in enzymatically active neutral protein and alkaline protease (subtilisin). The construction of strain BG2036 is described in detail in US Patent 5,264,366. Other host cells for expressing protein include *Bacillus subtilis* I168 (also described in US Patent 4,760,025 (RE 34,606) and US Patent 5,264,366) as well as any suitable *Bacillus* strain such as *B*. *licheniformis, B. lentus,* etc.

Host cells are transformed or transfected with vectors constructed using recombinant DNA techniques. These techniques can be found In any molecular biology practice guide, for example, Sambrook et al. Molecular Cloning - A Laboratory Manual (2nd ed.) Vol. 1-3, Cold Springs Harbor Publishing (1989) ("Sambrook"); and Current Protocols in Molecular Biology, Ausubel et al.(eds.), Current Protocols, a joint venture between Greene Publishing Associates, Inc. and John Wiley & Sons, Inc., (1997 Supplement) ("Ausubel"). Such transformed host cells are capable of either replicating vectors encoding the protein variants or expressing the desired protein variant. In the case of vectors which encode the pre- or prepro-form of the protein variant, such variants, when expressed, are typically secreted from the host cell into the host cell medium.

"Operably linked", when describing the relationship between two DNA regions, simply means that they are functionally related to each other. For example, a presequence is operably linked to a peptide if it functions as a signal sequence, participating in the secretion of the mature form of the protein most probably involving cleavage of the signal sequence. A promoter is operably linked to a coding sequence if it controls the transcription of the sequence; a ribosome binding site is operably linked to a coding sequence if it is positioned so as to permit translation.

The genes encoding the naturally-occurring precursor protein may be obtained in accord with the general methods known to those skilled in the art. The methods generally comprise synthesizing labeled probes having putative sequences encoding regions of the protein of interest, preparing genomic libraries from organisms expressing the protein, and screening the libraries for the gene of interest by hybridization to the probes. Positively hybridizing clones are then mapped and sequenced.

"Hybridization" is used to analyze whether a given DNA fragment or gene corresponds to a DNA sequence described herein and thus falls within the scope of the present invention. Samples to be hybridized are electrophoresed through an agarose gel (for example, 0.8% agarose) so that separation of DNA fragments can be visualized by size. DNA fragments are typically visualized by ethidium bromide staining. The gel may be briefly rinsed in distilled H2O and subsequently depurinated in an appropriate solution (such as, for example, 0.25M HCl) with gentle shaking followed by denaturation for 30 minutes (in, for example, 0.4 M NaOH) with gentle shaking. A renaturation step may be included, in which the gel is placed in 1.5 M NaCl, 1MTris, pH 7.0 with gentle shaking for 30 minutes.

The DNA should then be transferred onto an appropriate positively charged membrane, for example, Maximum Strength Nytran Plus membrane (Schleicher & Schuell, Keene, N.H.), using a transfer solution (such as, for example, 6XSSC (900 mM NaCl, 90 mM trisodium citrate). Once the transfer is complete, generally after about 2 hours, the membrane is rinsed in e.g., 2X SSC (2X SSC =300 mM NaCl, 30 mM trisodium citrate) and air dried at room temperature. The membrane should then be prehybridized (for approximately 2 hours or more) in a suitable prehybridization solution (such as, for example, an aqueous solution containing per 100 mL: 20-50 mL formamide, 25 mL of 20X SSPE (1X SSPE = 0.18 M NaCl, 1 mM EDTA, 10 mM NaH2PO4, pH 7.7), 2.5 mL of 20% SDS, and 1 mL of 10 mg/mL sheared herring or salmon sperm DNA). As would be known to one of skill in the art, the amount of formamide in the prehybridization solution may be varied depending on the nature of the reaction obtained according to routine methods. Thus, a lower amount of formamide may result in more complete hybridization in terms of identifying hybridizing molecules than the same procedure using a larger amount of formamide. On the other hand, a strong hybridization band may be more easily visually identified by using more formamide.

A DNA probe that is complementary or is nearly complementary to the DNA sequence of interest and is generally between 100 and 1000 bases in length is labeled (using, for example, the Megaprime labeling system according to the instructions of the manufacturer) to incorporate 32P in the DNA. The labeled probe is denatured by heating to 95°C for 5 minutes and immediately added to the membrane and prehybridization solution. The hybridization reaction should proceed for an appropriate time and under appropriate conditions, for example, for 18 hours at 37°C with gentle shaking or rotating. The membrane is rinsed (for example, in 2X SSC/0.3% SDS) and then washed in an appropriate wash solution with gentle agitation. The stringency desired will be a reflection of the conditions under which the membrane (filter) is washed.

Specifically, the stringency of a given reaction (*i*.*e*., the degree of homology necessary for successful hybridization) will depend on the washing conditions to which the filter is subjected after hybridization. "Low-stringency" conditions as defined herein will comprise washing a filter with a solution of 0.2X SSC/0.1 % SDS at 20°C for 15 minutes. "High-stringency" conditions comprise a further washing step comprising washing the filter a second time with a solution of 0.2X SSC/0.1 % SDS at 37°C for 30 minutes.

After washing, the membrane is dried and the bound probe detected. If 32P or another radioisotope is used as the labeling agent, the bound probe can be detected by autoradiography. Other techniques for the visualization of other probes are well-known to those of skill. The detection of a bound probe indicates a nucleic acid sequence has the desired homology and is encompassed within this invention.

The cloned protein is then used to transform a host cell in order to express the protein. The protein gene is then ligated into a high copy number plasmid. This plasmid replicates in hosts in the sense that it contains the well-known elements necessary for plasmid replication: a promoter operably linked to the gene in question (which may be supplied as the gene's own homologous promoter if it is recognized, *i*.*e*., transcribed, by the host), a transcription termination and polyadenylation region (necessary for stability of the mRNA transcribed by the host from the protein gene in certain eucaryotic host cells) which is exogenous or is supplied by the endogenous terminator region of the protein gene and, desirably, a selection gene such as an antibiotic resistance gene that enables continuous cultural maintenance of plasmid-infected host cells by growth in antibiotic-containing media. High copy number plasmids also contain an origin of replication for the host, thereby enabling large numbers of plasmids to be generated in the cytoplasm without chromosomal limitations. However, it is within the scope herein to integrate multiple copies of the protein gene into host genome. This is facilitated by procaryotic and eucaryotic organisms which are particularly susceptible to homologous recombination.

In one embodiment, the gene can be a natural gene such as that from *B lentus* or *B*. *amyloliquefaciens.* Alternatively, a synthetic gene encoding a naturally-occurring or mutant precursor protein may be produced. In such an approach, the DNA and/or amino acid sequence of the precursor protein is determined. Multiple, overlapping synthetic single-stranded DNA fragments are thereafter synthesized, which upon hybridization and ligation produce a synthetic DNA encoding the precursor protein. An example of synthetic gene construction is set forth in Example 3 of US Patent 5.204.015.

Once the naturally-occurring or synthetic precursor protein gene has been cloned, a number of modifications are undertaken to enhance the use of the gene beyond synthesis of the naturally-occurring precursor protein. Such modification include the production of recombinant proteins as disclosed in US Patent 4,760,025 (RE 34,606) and EPO Publication No. 0 251446 and the production of protein variants described herein.

The following cassette mutagenesis method may be used to facilitate the construction of the protein variants of the present invention, although other methods may be used. First, the naturally-occurring gene encoding the protein is obtained and sequenced in whole or in part. Then the sequence is scanned for a point at which it is desired to make a mutation (deletion, insertion or substitution) of one or more amino acids In the encoded enzyme. The sequences flanking this point are evaluated for the presence of restriction sites for replacing a short segment of the gene with an oligonucleotide pool which when expressed will encode various mutants. Such restriction sites are preferably unique sites within the protein gene so as to facilitate the replacement of the gene segment. However, any convenient restriction site which is not overly redundant in the protein gene may be used, provided the gene fragments generated by restriction digestion can be reassembled In proper sequence. If restriction sites are not present at locations within a convenient distance from the selected point (from 10 to 15 nucleotides), such sites are generated by substituting nucleotides In the gene in such a fashion that neither the reading frame nor the amino acids encoded are changed in the final construction. Mutation of the gene in order to change its sequence to conform to the desired sequence is accomplished by M13 primer extension in accord with generally known methods. The task of locating suitable flanking regions and evaluating the needed changes to arrive at two convenient restriction site sequences is made routine by the redundancy of the genetic code, a restriction enzyme map of the gene and the large number of different restriction enzymes. Note that if a convenient flanking restriction site is available, the above method need be used only in connection with the flanking region which does not contain a site.

Once the naturally-occurring DNA or synthetic DNA is cloned, the restriction sites flanking the positions to be mutated are digested with the cognate restriction enzymes and a plurality of end termini-complementary oligonucleotide cassettes are ligated into the gene. The mutagenesis is simplified by this method because all of the oligonucleotides can be synthesized so as to have the same restriction sites, and no synthetic linkers are necessary to create the restriction sites.

In one aspect of the invention, the objective is to secure a variant protein having altered allergenic potential as compared to the precursor protein, since decreasing such potential enables safer use of the enzyme. While the instant invention is useful to lower allergenic potential, the mutations specified herein may be utilized in combination with mutations known in the art to result altered thermal stability and/or altered substrate specificity, modified activity or altered alkaline stability as compared to the precursor.

The methods of the present invention may be directed to altering the capability of the T-cell epitope which includes residue positions 170-173 in *Bacillus lentus* to induce T-cell proliferation. Modification may be made to either one or all of R170D, Y171Q and/or N173D. Similarly, as discussed in detail above, it is believed that the modification of the corresponding residues in any protein will result in a the neutralization of a key T-cell epitope in that protein. Thus, in combination with the presently disclosed mutations in the region corresponding to amino acid residues 170-173, substitutions at positions corresponding to N76D/S103A/V104I/G159D optionally in combination with one or more substitutions selected from the group consisting of positions corresponding to V68A, T213R, A232V, Q236H, Q246R, and T260A of *Bacillus amyloliquefaciens* subtilisin may be used, In addition to decreasing the allergenic potential of the variant protein of the invention, to modulate overall stability and/or proteolytic activity of the enzyme. Similarly, these substitutions may be combined with mutation at the Asparagine (N) in *Bacillus lentus* subtilisin at equivalent position +76 to Aspartate (D) in combination with the mutations S103A/V104I/G159D and optionally in combination with one or more substitutions selected from the group consisting of positions corresponding to V68A, T213R, A232V, Q236H, Q245R, and T260A of *Bacillus amyloliquefaciens* subtilisin, to produce enhanced stability and/or enhanced activity of the resulting mutant enzyme.

Particular variants include the following specific combinations of substituted residues corresponding to positions:
N76D/S103A/V104I/G159D/K170D/Y171Q/S173D;
V68A/N76D/S103A/V104I/G159D/K170D/Y171O/S173D/Q236H;
V68A/N76D/S103A/V104I/G159D/K170D/Y171Q/S173D/Q236H/Q245R;
V68A/N76D/S103A/V104I/G159D/K170D/Y171Q/S173D/A232V/Q236H/Q245R; and
V68A/N76D//S103A/V104I/G159D/K170D/Y171Q/S173D/T213R/A232V/Q236H/
Q245R/T260A of *Bacillus amyloliquefaciens* subtilisin. These substitutions are preferably made In *Bacillus lentus* (recombinant or native-type) subtilisin, although the substitutions may be made in any *Bacillus* protein.

Based on the screening results obtained with the variant proteins, the noted mutations noted above in *Bacillus amyloliquefaciens* subtilisin are important to the proteolytic activity, performance and/or stability of these enzymes and the cleaning or wash performance of such variant enzymes.

Many of the protein variants are useful in formulating various detergent compositions. A number of known compounds are suitable surfactants useful in compositions comprising the protein mutants. These include nonionic, anionic, cationic, anionic or zwitterionic detergents, as disclosed in US 4,404,128 to Barry J. Anderson and US 4,261,868 to Jiri Flora, et al. A suitable detergent formulation is that described in Example 7 of US Patent 5,204,015. The art is familiar with the different formulations which can be used as cleaning compositions. In addition to typical cleaning compositions, it is readily understood that the protein variants may be used for any purpose that native or wild-type proteins are used. Thus, these variants can be used, for example, in bar or liquid soap applications, dishcare formulations, contact lens cleaning solutions or products, peptide hydrolysis, waste treatment, textile applications, as fusion-cleavage enzymes in protein production, etc. The varlants may comprise, in addition to decreased allergenicity, enhanced performance in a detergent composition (as compared to the precursor). As used herein, enhanced performance in a detergent is defined as increasing cleaning of certain enzyme sensitive stains such as grass or blood, as determined by usual evaluation after a standard wash cycle.

Proteins, particularly proteases can be formulated into known powdered and liquid detergents having pH between 6.5 and 12.0 at levels of about .01 to about 5% (preferably 1% to 5%) by weight. These detergent cleaning compositions can also include other enzymes such as known proteases, amylases, cellulases, lipases or endoglycosidases, as well as builders and stabilizers.

The addition of proteins, particularly proteases to conventional cleaning compositions does not create any special use limitation. In other words, any temperature and pH suitable for the detergent is also suitable for the present compositions as long as the pH is within the above range, and the temperature is below the described protein's denaturing temperature. In addition, proteins can be used in a cleaning composition without detergents, again either alone or in combination with builders and stabilizers.

Variant proteins can be included in animal feed such as part of animal feed additives as described in, for example, US 5,612,055; US 5,314,692; and US 5,147,642.

Variant proteins can be used to treat for example slik or wool as described in publications such as RD 216,034; EP 134,267; US 4,533,359; and EP 344,259.

The variants can be screened for proteolytic activity according to methods well known in the art. One of skill will readily recognize that the uses of proteins will be determined, in large part, on the immunological properties of the proteins. For example, enzymes that exhibit reduced allergenicity can be used in cleaning compositions. "Cleaning compositions" are compositions that can be used to remove undesired compounds from substrates, such as fabric, dishes, contact lenses, other solid substrates, hair (shampoos), skin (soaps and creams), etc. Proteins, in particular, cellulases, proteases, and amylases, with reduced allergenicity can also be used in the treatment of textiles. "Textile treatment" comprises a process wherein textiles, individual yarns or fibers that can be woven, felted or knitted into textiles or garments are treated to effect a desired characteristic. Examples of such desired characteristics are "stone-washing", depilling, dehairing, desizing, softening, and other textile treatments well known to those of skill in the art.

The methods of the invention may be applied to therapeutic proteins against which individuals mount an immune response in particular, individuals who lack endogenous production of the protein are susceptible to forming neutralizing antibodies and become refractile to treatment. Likewise, modifications of a protein may introduce new epitopes that are potentially immunogeneic. Methods of the invention can be used to identify and modify epitopes In , e.g., human Factor VIII, to prevent neutralizing responses.

The pharmaceutical compositions can be prepared in various forms, such as granules, tablets, pills, suppositories, capsules, suspensions, salves, lotions and the like. Pharmaceutical grade organic or inorganic carriers and/or diluents suitable for oral and topical use can be used to make up compositions containing the therapeutically_active compounds. Diluents known to the art include aqueous media, vegetable and animal oils and fats. Stabilizing agents, wetting and emulsifying agents, salts for varying the osmotic pressure or buffers for securing an adequate pH value, and skin penetration enhancers can be used as auxiliary agents. The pharmaceutical compositions may also include one or more of the following: carrier proteins such as serum albumin; buffers; fillers such as microcrystalline cellulose, lactose, com and other starches; binding agents; sweeteners and other flavoring agents; coloring agents; and polyethylene glycol. Additives are well known in the art, and are used in a variety of formulations.

The following is presented by way of example and is not to be construed as a limitation to the scope of the claims.

### EXAMPLES

### Example 1

### Assay for the Identification of Peptide T-Cell Epitopes Using Naïve Human T-Cells

Fresh human peripheral blood cells were collected from "naïve" humans, *i.e*., persons not known to be exposed to or sensitized to *Bacillus lentus* protease, for determination of antigenic epitopes In protease from *Bacillus lentus* and human subtilisin. Naïve humans is intended to mean that the individual is not known to have been exposed to or developed a reaction to protease in the past. Peripheral mononuclear blood cells (stored at room temperature, no older than 24 hours) were prepared for use as follows: Approximately 30 mls of a solution of buffy coat preparation from one unit of whole blood was brought to 50 ml with Dulbecco's phosphate buffered solution (DPBS) and split Into two tubes. The samples were underlaid with 12.5 ml of room temperature lymphoprep density separation media (Nycomed density 1.077 g/ml). The tubes were centrifuged for thirty minutes at 600G. The interface of the two phases was collected, pooled and washed in DPBS. The cell density of the resultant solution was measured by hemocytometer. Viability was measured by trypan blue exclusion.

From the resulting solution, a differentiated dendritic cell culture was prepared from the peripheral blood mononuclear cell sample having a density of 108 cells per 75 ml culture flask in a solution as follows:
(1) 50 ml of serum free AIM V media (Gibco) was supplemented with a 1:100 dilution beta-mercaptoethanol (Gibco). The flasks were laid flat for two hours at 37°C in 5% C02 to allow adherence of monocytes to the flask wall.
(2) Differentiation of the monocyte cells to dendritic cells was as follows:
   nonadherent cells were removed and the resultant adherent cells (monocytes) combined with 30 ml of AIM V, 800 units/ml of GM-CSF (Endogen) and 500 units/ml of IL-4 (Endogen);
   the resulting mixture was cultured for 5 days under conditions at 37°C in 5% CO2. After five days, the cytokine TNFa (Endogen) was added to 0.2 units/ml, and the cytokine IL-1a (Endogen) was added to a final concentration of 50 units/ml and the mixture incubated at 37°C in 5% CO2 for two more days.
(3) On the seventh day, Mitomycin C was added to a concentration of 50 microgram/ml was added to stop growth of the now differentiated dendritic cell culture. The solution was incubated for 60 minutes at 37°C in 5% CO2. Dendritic cells were collected by gently scraping the adherent cells off the bottom of the flask with a cell scraper. Adherent and non-adherent cells were then centrifuged at 600G for 5 minutes, washed in DPBS and counted.
(4) The prepared dendritic cells were placed into a 96 well round bottom array at 2x104/well in 100 microliter total volume of AIM V media.

CD4+ T cells were prepared from frozen aliquots of the peripheral blood cell samples used to prepare the dendritic cells using the human CD4+ Cellect Kit (Biotex) as per the manufacturers instructions with the following modifications: the aliquots were thawed and washed such that approximately 108 cells will be applied per Cellect column; the cells were resuspended in 4 ml DPBS and 1 ml of the Cell reagent from the Cellect Kit, the solution maintained at room temperature for 20 minutes. The resultant solution was centrifuged for five minutes at 600G at room temperature and the pellet resuspended in 2 ml of DPBS and applied to the Cellect columns. The effluent from the columns was collected in 2% human serum in DPBS. The resultant CD4+ cell solution was centrifuged, resuspended in AIMV media and the density counted.

The CD4+ T-cell suspension was resuspended to a count of 2x106/ml in AIM V media to facilitate efficient manipulation of the 96 well plate.

Peptide antigen is prepared from a 1 M stock solution in DMSO by dilution in AIM V media at a 1:10 ratio. 10 microliters of the stock solution is placed in each well of the 96 well plate containing the differentiated dendritic cells. 100 microliter of the diluted CD4+ T-cell solution as prepared above is further added to each well. Useful controls include diluted DMSO blanks, and tetanus toxoid positive controls.

The final concentrations in each well, at 210 microliter total volume are as follows:
2x104 CD4+
2x105 dendtritic cells (R:S of 10:1)
5 mM peptide

### Example 2

### Identification of T-Cell Epitopes in Protease from Bacillus lentus and Human subtilisin

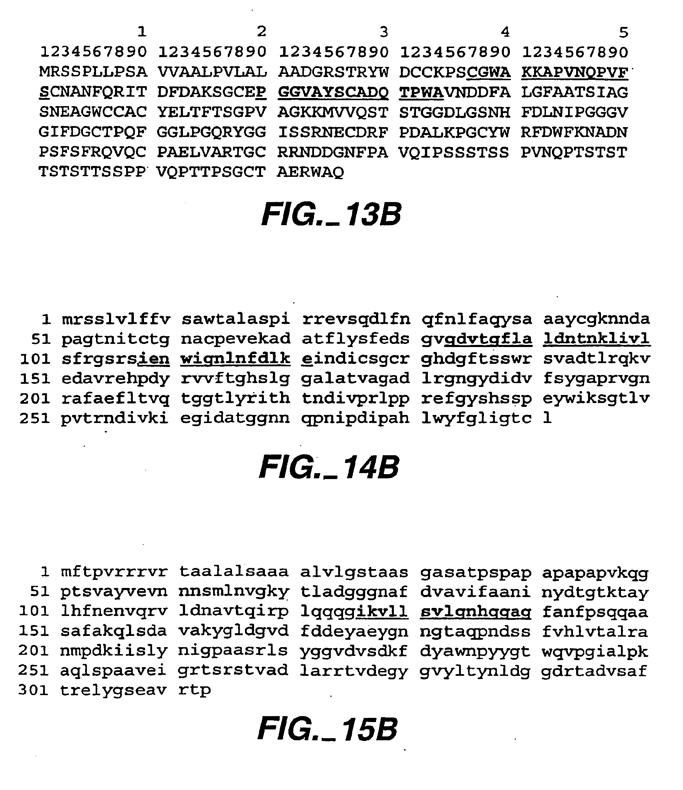

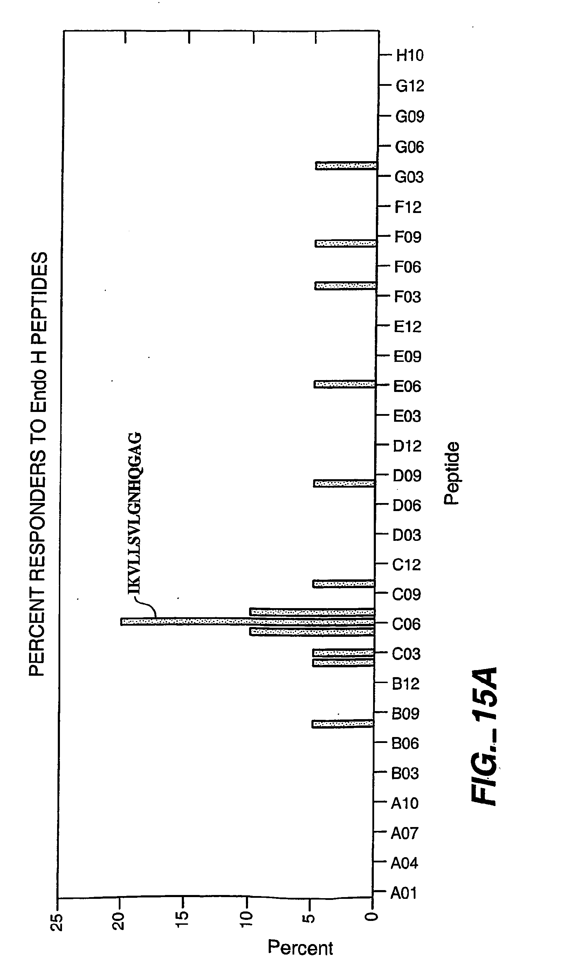

Peptides for use in the assay described in Example 1 were prepared based on the *Bacillus lentus* and human subtilisin amino acid sequence. Peptide antigens were designed as follows. From the full length amino acid sequence of either human subtilisin or *Bacillus lentus* protease provided in Figure 1, 15mers were synthetically prepared, each 15mer overlapping with the previous and the subsequent 15mer except for three residues.

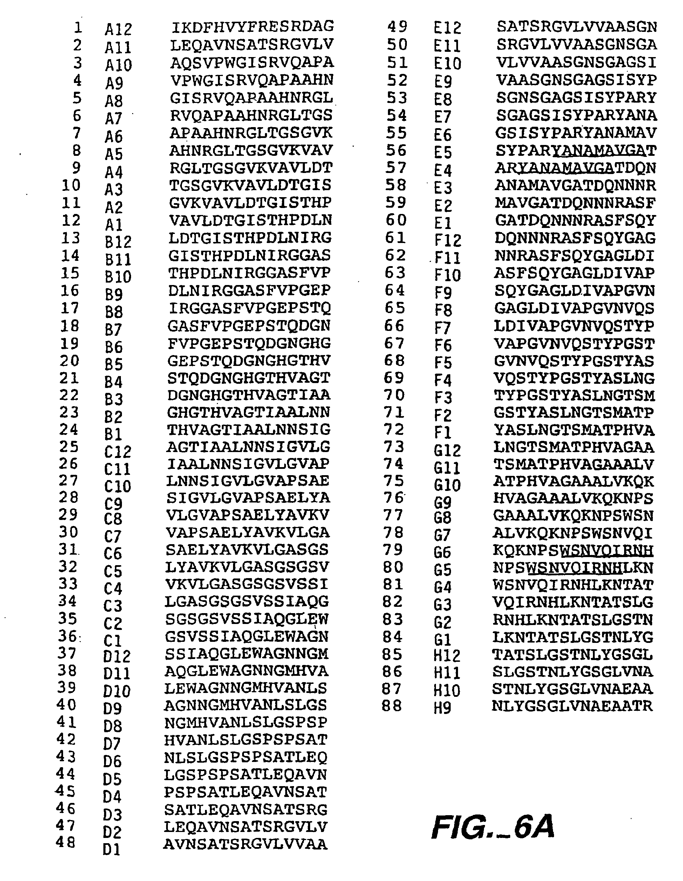

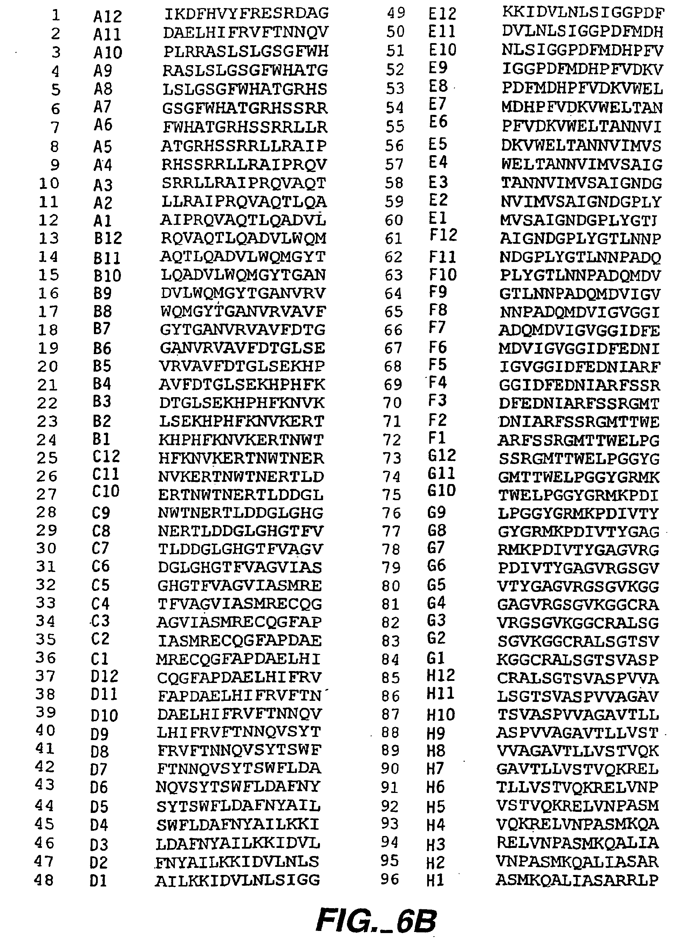

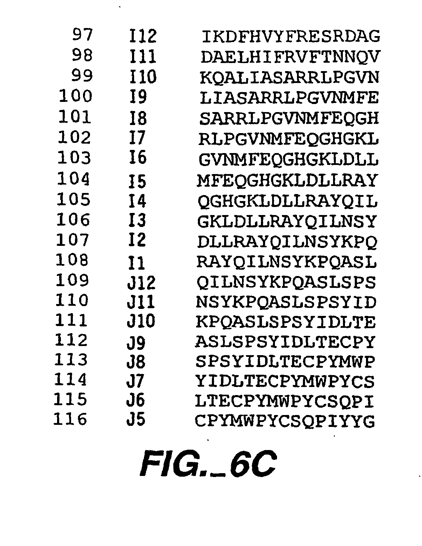

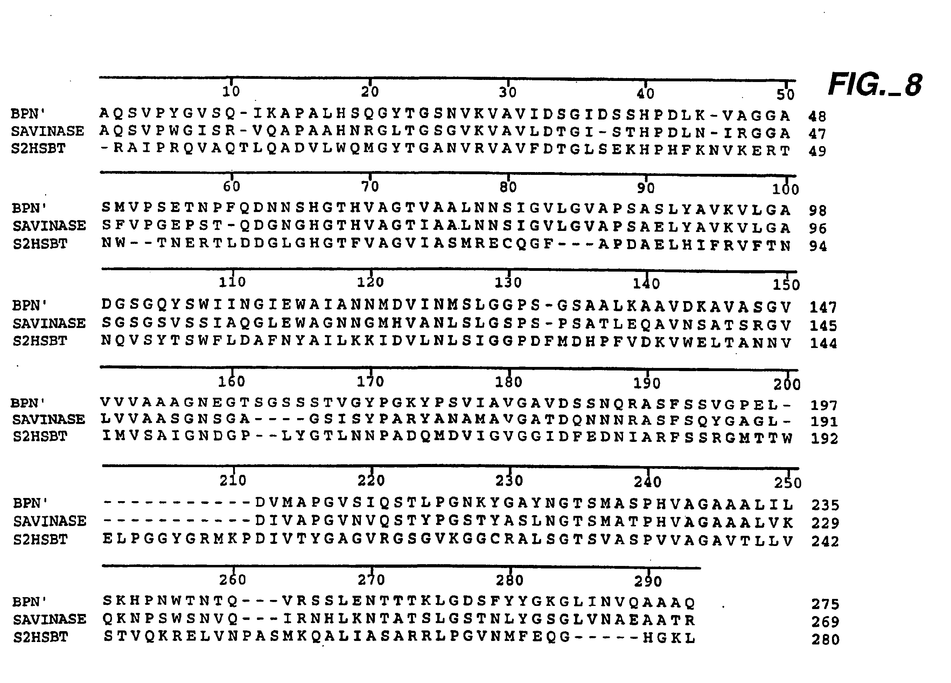

Peptides used correspond to amino acid residue strings in *Bacillus lentus* as provided in Figure 8, and peptides correspond to amino acid residues in human subtilisin as provided in Figure 7. The peptides used corresponding to the proteases is provided in Fig. 6. All tests were performed at least in duplicate. All tests reported displayed robust positive control responses to the antigen tetanus toxoid. Responses were averaged within each experiment, then normalized to the baseline response. A positive event was recorded if the response was at least 3 times the baseline response.

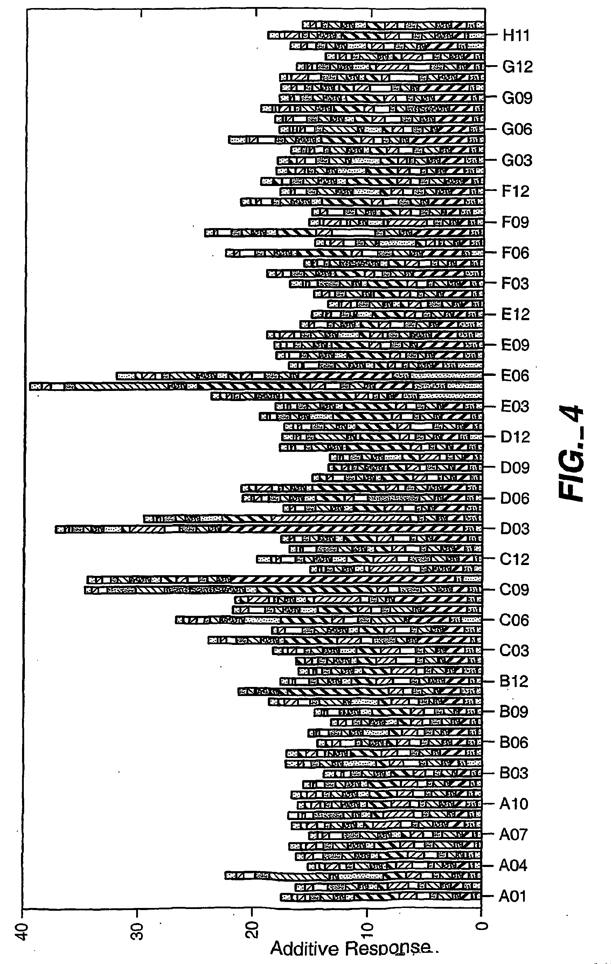

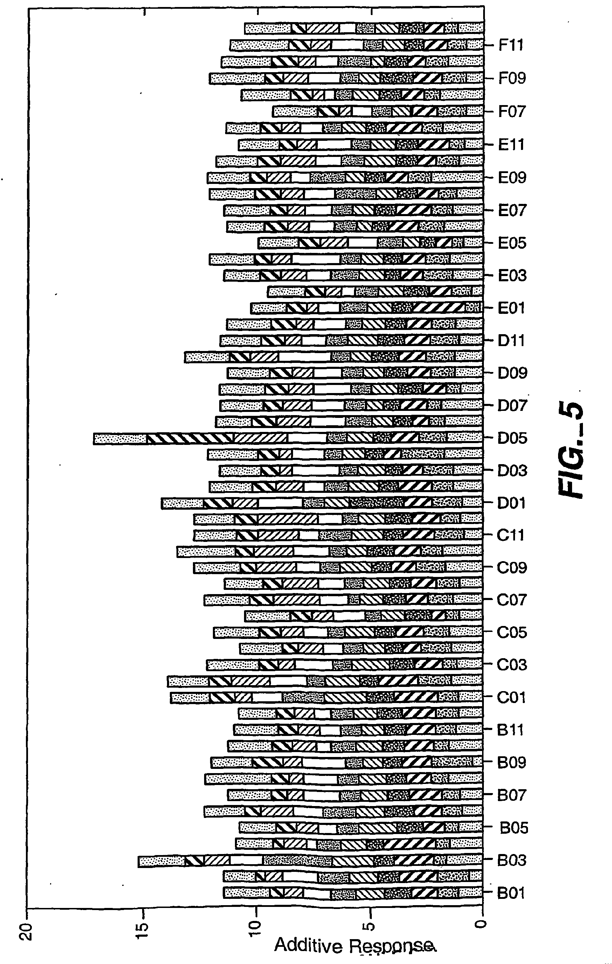

The immunogenic response (*i*.*e*., T-cell proliferation) to the prepared peptides from human subtilisin and *Bacillus lentus* was tallied and is provided in Figures 4 and 5, respectively. T-cell proliferation was measured by the incorporated tritium method. The results shown in Figures 4 and 5 as a comparison of the immunogenic additive response in 10 individuals (Figure 4) and 16 individuals (Figure 5) to the various peptides. Response is indicated as the added response wherein 1.0 equals a baseline response for each sample. Thus, in Figure 4, a reading of 10.0 or less is the baseline response and in Figure 5 a reading of 16.0 or less the baseline response. The greater the response, the more potent the T-cell epitope is considered.

As indicated in Figures 4 and 5, the immunogenic response of the naïve blood samples from unsensitized individuals showed a marked allergenic response at the peptide fragment from *Bacillus lentus* corresponding to residues 170-173 of *Bacillus amyloliquefaciens* protease. As expected, the corresponding fragment in human subtilisin evokes merely baseline response.

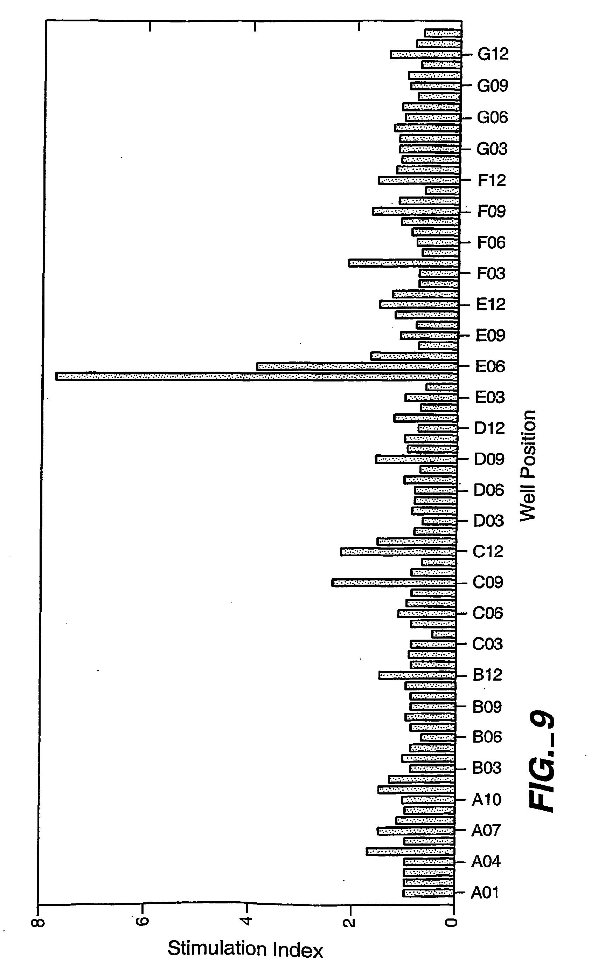

Fig. 9 shows the T-cell response to peptides derived from *Bacillus lentus* protease in a sample taken from an individual known to be hypersensitive to *Bacillus lentus* protease. Peptide E05 represents the region corresponding to 170-173 in protease from *Bacillus amyloliquefaciens.* As shown in Fig. 9, the hypersensitive individual was highly responsive to the T-cell epitope represented by the peptide E05. This result confirms that, by practicing the assay according to the invention, it is possible to predict the major epitopes identified by the T-cells of a hypersensitive individual.

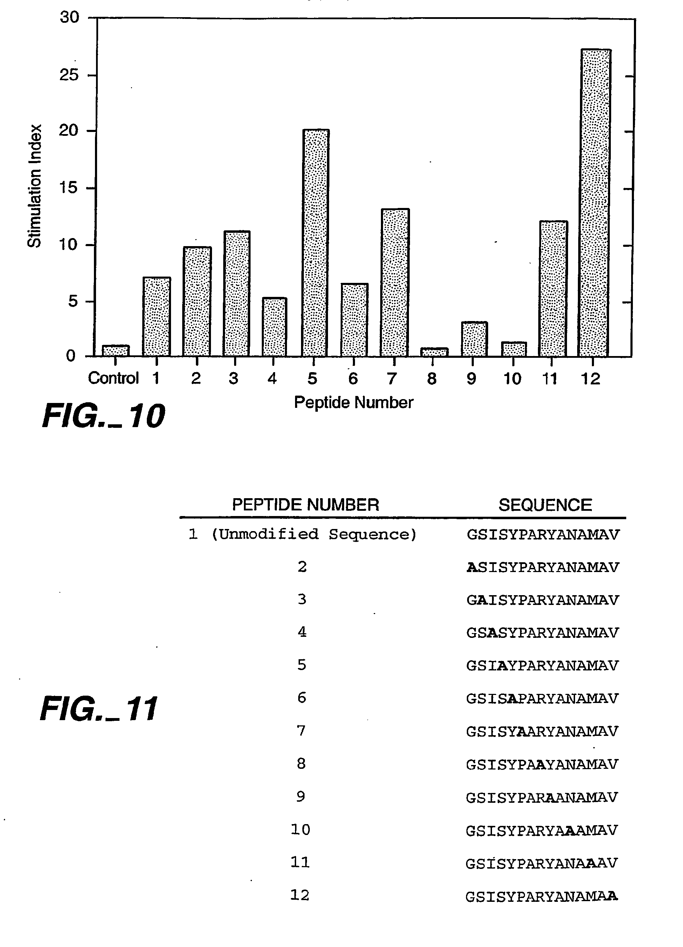

Fig.10 shows the T-cell response to various alanine substitutions in the E05 peptide derived from *Bacillus lentus* protease in a sample taken from an individual known to be hypersensitive to *Bacillus lentus* protease. Alanine substitutions were used as substitutions for the purpose of determining the role of any specific residue within the epitope. The legend of Figure 10 refers to the position of the peptide in which an alanine was substituted, i.e., in peptide E06 (sequence GSISYPARYANAMAV), G to A=2, S to A=3, I to A=4, S to A=5, Y to A=6, P to A=7, R to A= 8, Y to A = 9, N to A = 10, M to A = 11 and V to A = 12. As indicated in Figure 10, substitution of either of the residues R170A, Y171A and/or N173A in protease from *Bacillus lentus* results in dramatically reduced response in the hypersensitive individual's blood sample.

From these results, it is apparent that the residues 170, 171 and 173 are largely responsible for the initiation of allergic reaction within the protease from *Bacillus lentus.*

### Example 3

### Identification of T-Cell Epitopes in Cellulase from Humicola insolens (Carezyme®)

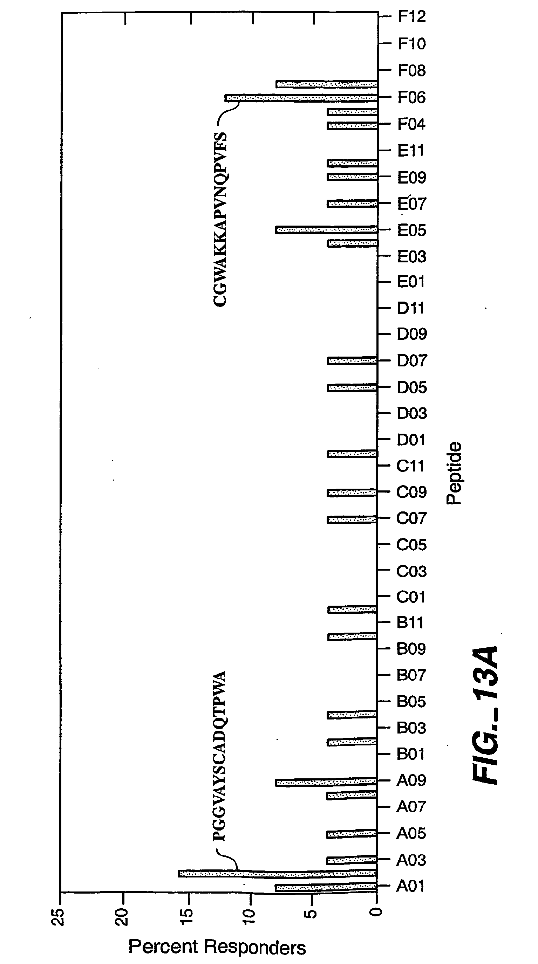

The procedure described above was performed on peptides derived from a cellulase from *Humicola insolens* (Carezyme® from Novo Nordisk). As can be seen from Figure 13, 2 T-cell epitopes were discovered, A01 and F06.

### Example 4

### Identification of T-Cell Epitopes in Lipase from Thermomyces Lanuginosa (Lipolase®)

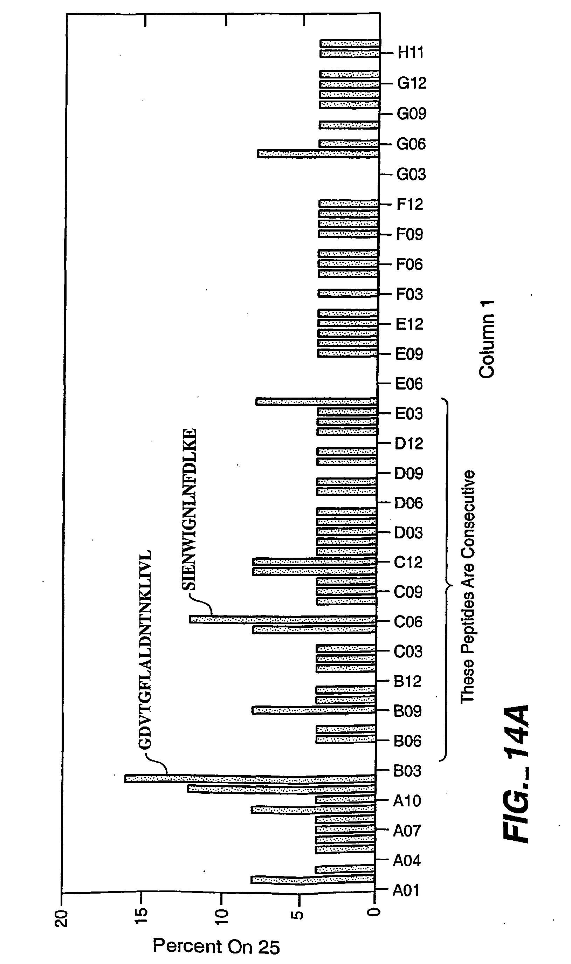

The procedure described in Example 2 was performed on peptides derived from a lipase from *Thermomyces lanuginosa* (Lipolase ® from Novo Nordisk). As can be seen from Figure 14, two T-cell epitopes were discovered, A12 and C06. Peptide E03 effected slightly increased T-cell proliferation in the naïve donors, however, this peptide is consecutive to A12 and they represent one epitope. In this regard, the skilled artisan understands that the length of the epitopes can be varied, and the precise potency of the epitope, naturally occuring or mutated can be determined by the methods herein.

### Example 5

### Identification of T-Cell Epitopes in Endoglucanase H from Streptomyces plicatus

The procedure described in Example 2 was performed on peptides derived from endoglucanase H from *Streptomyces plicatus.* As can be seen from Figure 15, a single T-cell epitope was discovered, C06.

### Example 6

### Identification of T-Cell Epitopes in a Protease Hybrid (GG36-BPN')

After determining the location of a T-cell epitope, a protease hybrid was constructed using established protein engineering techniques. The hybrid was constructed so that a highly allergenic amino acid sequence of the protein was replaced with a corresponding sequence from a less allergenic homolog. In this instance, the first 122 amino acids of the protease were derived from GG36, and the remaining amino acid sequence was derived from BPN'.

The hybrid was first tested from a 100 ppm sample in North American condition in 24 well assay at .5 ppm, superfixed swatches, liquid (Tide KT) at .5 in 24 well assay with 3K swatches, and in the N'N-dimethyl Casein Assay, 5 g/l DMC in NA detergent, TNBS dectection method.

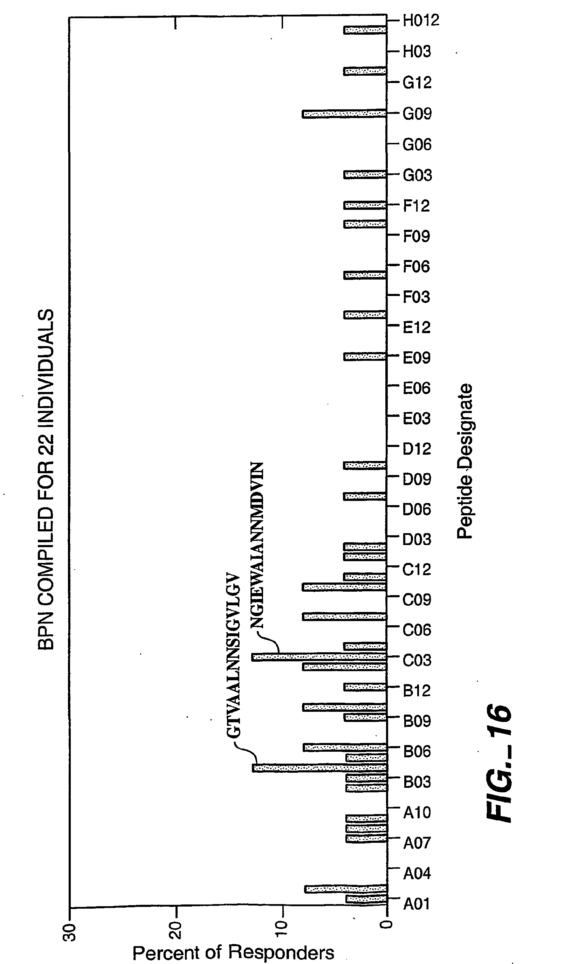

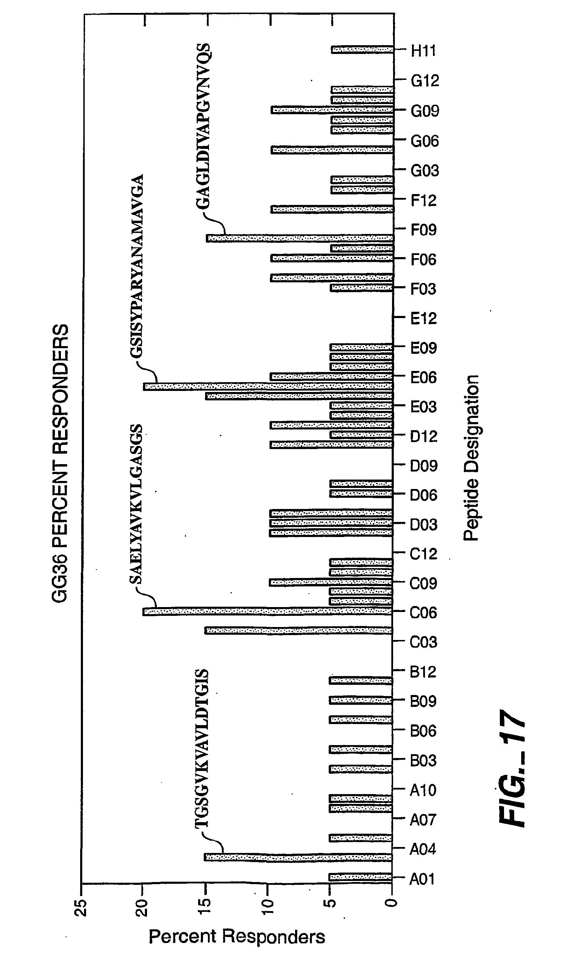

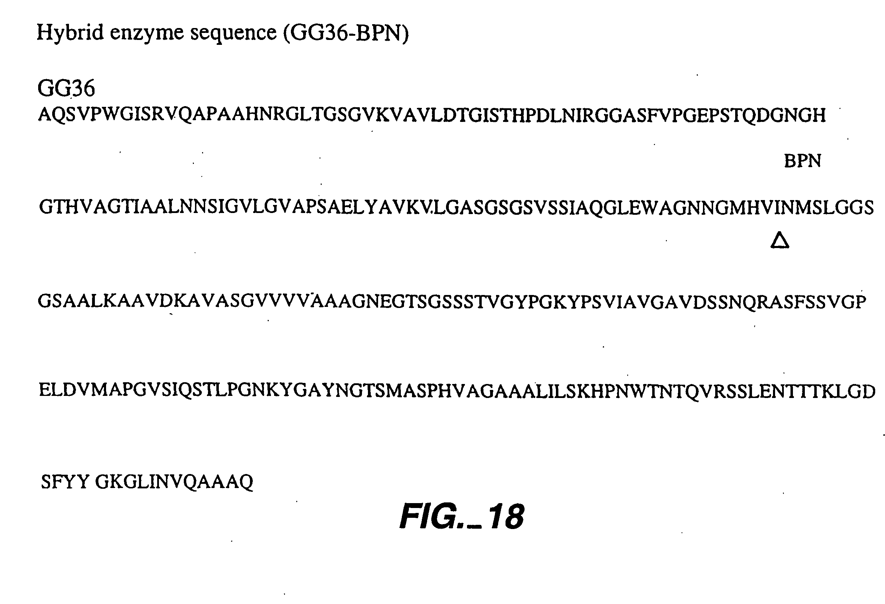

The results are shown in Figures 16, 17 and 18.

### Example 7

### Identification of a Naturally Occuring Low Immunogenic Protein

Using the methods herein, proteinase K was identified as producing a lower immunogenic response than other commercially available proteases. Proteinase K as identified herein is from Tritirachium Album limber. For a general description of proteases and methodologies, see, Mathew, C.G.P. Isolation of high molecular weight eukaryotic DNA, in Methods in Molecular Biology, vol. 2: Nucleic Acids (Walker, J.M.,ed.), Humana, Clifton, NJ, (1984) pp. 31-34.

### Example 8:

### T-cell Epitope Introduced into a Non-allergenic Protein

It has been found that *Bacillus amyloliquefaciens* subtilisin is comparatively non-immunogenic when tested in Hartley strain guinea pigs. A related protein from *Bacillus lentis* is highly immunogenic. We had previously defined functional T cell epitopes in the *B. lentis* molecule which were not found in the *B*. *amyloliquefaciens* molecule, even though the sequences of interest were highly homologous. In order to test the principle that the presence of a functional T cell epitope can control the relative levels of antibody production, we created a *B. lentis-like* T cell epitope in the *B. amyloliquefaciens* molecule. This change was accomplished by the substitution of a single amino acid in the *B. amyloliquefaciens* sequence. *B. amyloliquefaciens* subtilisin and the T cell epitope modified variant of *B*. *amyloliquefaciens* subtilisin were tested in a guinea pig model of immungenicity.

*B. lentis* and *B*. *amyloliquefaciens* subtilisin T cell epitope mapping: Guinea pigs were immunized with 20 µg/immunization of subtilisin from either *B*. *lentis* or *B*. *amyloliquefaciens.* Animals were immunized subcutaneously in adjuvant every two weeks for 10 to 12 weeks. A single cell suspension of guinea pig splenocytes was created from each animal's spleen. Cells were plated at 5 x 10⁵ splenocytes per well in round bottom 96 well plates. 15-mer peptides off-set by 3 amino acids were synthesized by Mimotopes. Peptides were resuspended to 1 mM in DMSO. Peptides were added to the cells at a final concentration of 5µM. Cultures were incubated for 5 days at 37 °, 5% CO₂. Wells were pulsed with 0.5 µCi tritiated thymidine, and allowed to incubate for an additional 18 hours. Wells were harvested, and thymidine incorporation assessed.

Two T cell epitopes were found in *B*. *lentis* subtilisin, and none were found in *B*. *amyloliquefaciens* subtilisin (>10 animals tested for these epitopes). The *B. lentis* T cell epitopes were found to comprise the following sequences:
IAALNNSIGVLGVAP (SEQ ID NO:237) and LEWAGNNGMHVANLSLGS (SEQ ID NO:238)

For SEQ ID NO:237, the similar sequence in *B. amyloliquefaciens* subtilisin is VAALNNSIGVLGVAP (SEQ ID NO:239). The similar region in *B*. *amyloliquefaciens* subtilisin for SEQ ID NO:238 was the much less homologous: IEWAIANNMDVINMSLG (SEQ ID NO:240).

SEQ ID NO:237 and the homologous region in the *B. amyloliquefaciens* subtilisin molecule (SEQ ID NO: 239) differ by one amino acid: In *B. lentis* subtilisin the first amino acid is an I, while it is a V in *B*. *amyloliquefaciens.* Therefore, we reasoned that if we changed the V in the *B*. *amyloliquefaciens* sequence to an I, we would create the *B*. *lentis* T cell epitope in the *B*. *amyloliquefaciens* backbone.

This molecule was created by standard molecular biological techniques, and was called *B*. *amyloliquefaciens* V72I. It was also known as GP002.

Guinea pig immunizations: Adult female Hartley guinea pigs were immunized with various doses of *B. amyloliquefaciens* subtilisin and GP002. The doses were 1, 5, 10, and 20 µg/dose. There were four animals for each dose. Animals were immunized subcutaneously with enzyme in Complete Freund's Adjuvant for the first immunization. All subsequent immunizations were performed in Incomplete Freund's adjuvant. Animals were immunized, and a serum sample taken, every two weeks.

ELI SA: A direct ELISA was performed. Costart EIA plates were coated with 10 µg/ml of the immunizing enzyme in PBS overnight at 4°C. Plates were washed and blocked with 1% BSA in PBS. Serum samples were diluted in 1% BSA/PBS, and incubated on the enzymes coated plates for 1 hour. Serum samples were washed out, and biotinylated anti-guinea pig IgG was added at a 1:10,000 dilution in 1% BSA/PBS. The secondary reagent was incubated for 1 hour. The wells were washed, and avidin conjugated horse radish peroxidase was added to the wells at a 1:1000 dilution in 1 % BSA/PBS. After 30 minutes, the substrate (ABTS) was added and the OD₄₀₅ was read after 30 minutes.

Calculation of titers: Background was subtracted from the OD readings, and the results plotted for each individual guinea pig. A linear regression analysis was performed on the linear portion of the curve. The titer value was calculated from the linear regression equation for an OD = 0.5. These individual titers were then averaged.

Two guinea pigs in the 10 µg dose of GP001 died at 2 weeks into the study. The data for the 10 µg dose was therefore thrown out.

Two results are immediately apparent: first, the GP002 variant increased the titers of antigen-specific antibody over the entire time course for the lower doses of enzymes; and the GP002 variant increased titers of antigen-specific antibody for all doses of enzymes in the earliest time points.

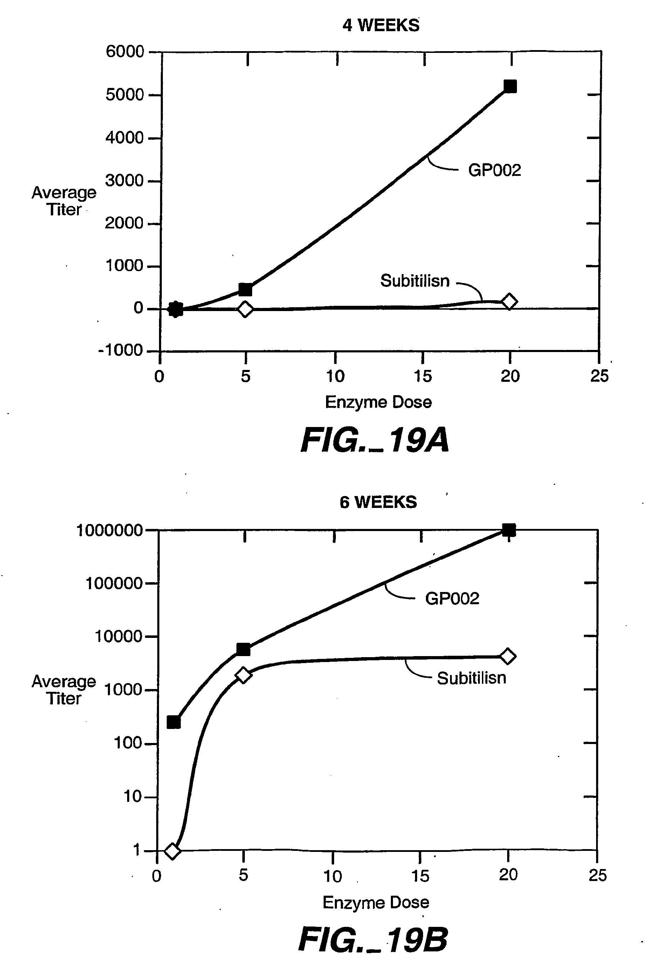

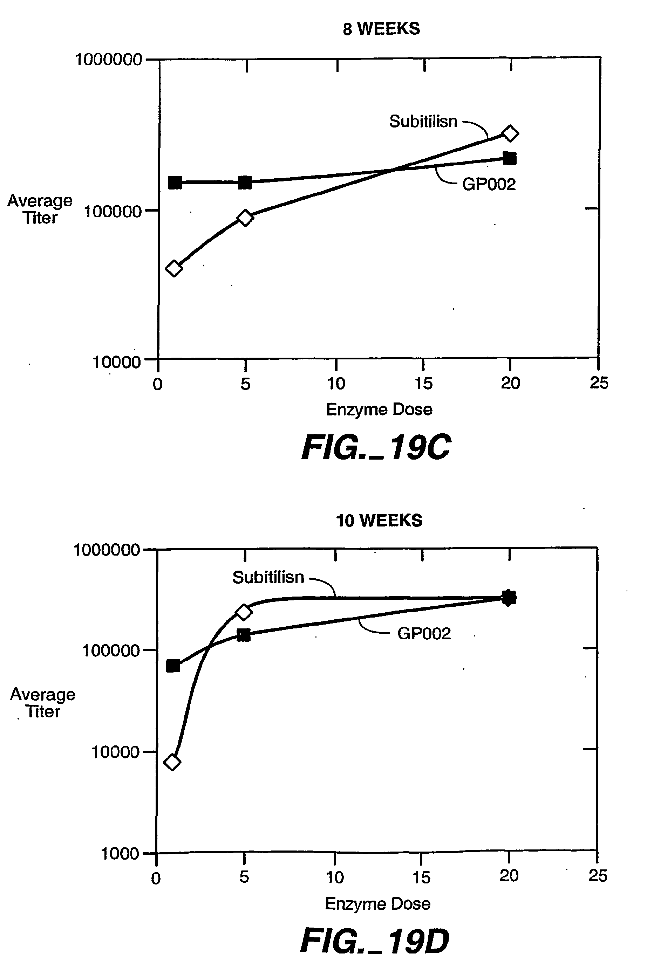

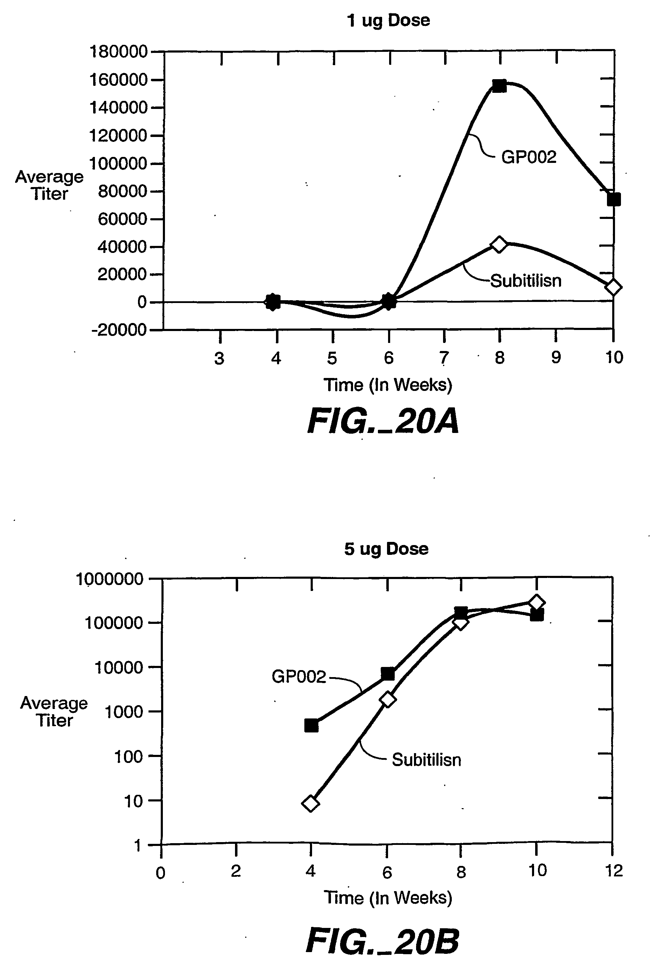

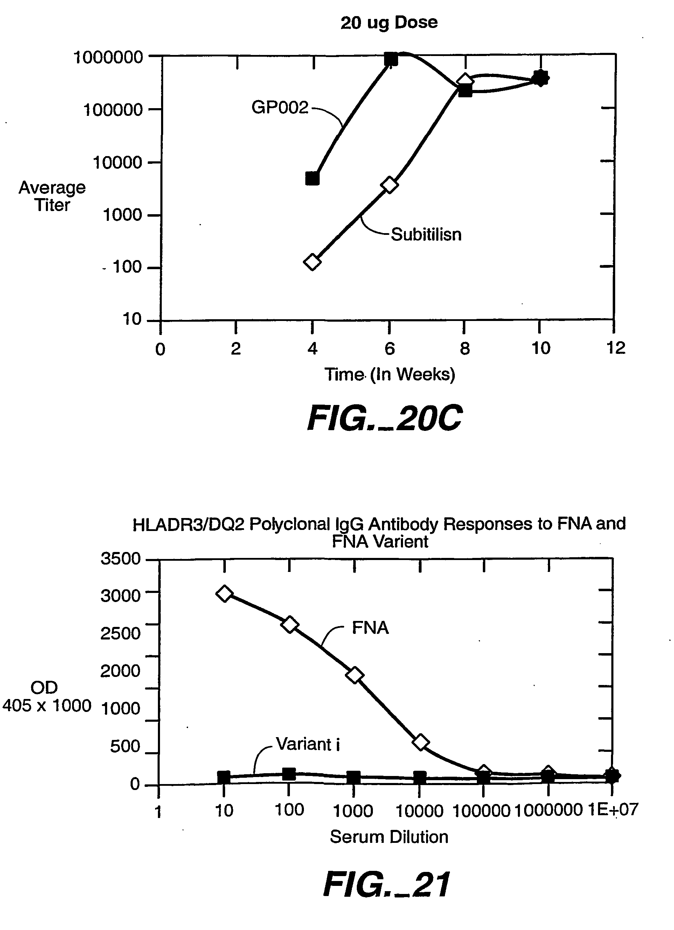

At the extended time points and for the higher doses, the difference between *B*. *amyloliquefaciens* subtilisin and its variant were no longer apparent. See Figures 19 and 20.

From the Figures it is apparent that a single change in the amino acid sequence of *B*. *amyloliquefaciens* subtilisin significantly altered its immunogenicity.

### Example 9

### Reduction of Allergenicity in Vivo

Given the ability to identity of human T cell epitopes, it is possible to modify their amino acid sequence to reduce activation of T cells and the subsequent immune response to the protein. However, to evaluate the *in vivo* effect of these changes, it is necessary to use an animal model that represents the ability of human HLA molecules to present the epitopes. For example, human T cell epitopes have been identified in the molecule BPN' in the regions 70-84 and 109-122 (see USSN 09/500,135, filed February 8, 2000; Figure 16).

Substitutions in the amino acid sequence of these motifs led to reduced T cell proliferation *in vitro* using human cells representing a broad range of human HLA haplotypes. In vitro binding assays using EBV transformed B cell lines demonstrated the peptides 70-84 and 109-123 bound to HLA DQ2 molecules. The substitutions that were found to reduce T-cell) proliferation were introduced into the coding sequence for FNA (BPN' with a Y217L substitution) for production of reduced immunogenic FNA variants.

Transgenic mice expressing human HLA genes have been used to study epitopes presented to the immune system *in vivo.* Although the responding immune cells are of mouse origin, there is a strong correlation between the epitopes recognized in humans and mice. However, a novel use of HLA transgenic mice is in the testing of variant proteins for reduced allergenic potential as a prediction of how human individuals will respond.

To demonstrate this effect, both FNA and the FNA variant containing amino acid changes in the epitopes 70-84 and 109-123 were used to immunize HLA DR3/DQ2 transgenic mice that had been backcrossed onto I-Ab knockout mice (lacking the expression of endogenous I-A class II molecules, referred to as C2D). Adult male HLA-DR3/DQ2/C2D mice were immunized with 50 µg of FNA or FNA Variant emulsified in Complete Freund's Adjuvant. The immunization was administered intraperitoneally. Two weeks later, the mice received another intraperitoneal immunization of 50 µg FNA or the Variant emulsified in incomplete Freund's Adjuvant. One week later, the mice were bled via the retro-orbital route, and the serum collected. Serum was assessed for antigen-specific IgG antibodies in a direct ELISA protocol. Briefly, 96 well flat-bottomed EIA plates were coated overnight with 10 µg/ml of denatured FNA. Plates were washed, blocked with 1% Fetal Calf Serum, and serum was titered out at 1:10 dilutions. The serum was washed out of the wells, and antigen-specific IgG was detected with horse radish peroxidase conjugated anti-mouse IgG. Results are presented as serum dilution versus average optical density (x 1000) in Table 1 and Figure 21.

**Table 1**

| **Dilution** | **FNA** | **FNA Variant** |
|---|---|---|
| 10 | 2937.5 | 88 |
| 100 | 2476 | 120 |
| 1000 | 1695 | 103 |
| 10000 | 641.5 | 80 |
| 100000 | 207 | 85 |
| 1000000 | 129.5 | 76 |
| 10000000 | 88.5 | 85 |

The results indicated the changes introduced into regions 70-84 and 109-123 significantly reduced the ability of DQ2 transgenic mice to mount a humoral response to the variant and provide a method for *in vivo* characterization of engineered proteins predicted with the methods of this invention to show reduced immunogenicity in humans.

## Claims

1. A method to determine the allergenic potential of an engineered protein comprising the steps of,
a) immunizing a first transgenic mouse with a protein of interest and immunizing a second transgenic mouse with an engineered protein wherein said engineered protein is a variant of said protein of interest and said protein of interest includes a T-cell epitope wherein the variant differs from the protein of interest by having an altered T cell epitope;
b) collecting serum of said first and said second immunized transgenic mice;
c) measuring the serum for antigen specific immunoglobulins; and
d) comparing the immunogenic response of said variant and said protein of interest; wherein the first transgenic mouse and second transgenic mouse are HLA DR3/DQ2, and wherein the variant and the protein of interest produce a different immunogenic response in said transgenic mice.

2. The method according to claim 1, wherein said protein of interest is an enzyme.

3. The method according to claim 2, wherein said enzyme is a protease.

4. The method according to claim 1, wherein the antigen specific immunoglobulin is IgG.

5. The method according to claim 1, wherein the HLA DR3/DQ2 transgenic mice have been backcrossed with mice lacking the expression of endogenous I-A class II molecules.

6. The method according to claim 1, wherein said T-cell epitope is altered with amino acid substitutions.

7. The method according to claim 1, wherein said T-cell epitope is altered by having a terminal portion of said protein of interest which includes said T-cell epitope replaced with a corresponding terminal portion of a homolog of said protein of interest wherein said homolog does not comprise a T-cell epitope identical to said replaced T-cell epitope.

8. The method according to claim 1, wherein said immunogenic response produced by the variant is less than the immunogenic response produced by the protein of interest.

9. The method according to claim 1, wherein said immunogenic response produced by the variant is more than the immunogenic response produced by the protein of interest.

10. A method according to claim 1 wherein said immunogenic response is predictive of the allergenic response in humans.

11. The method according to claim 10, wherein said protein of interest is a protease.

## Patentansprüche

1. Verfahren zur Bestimmung des allergenen Potenzials eines gentechnisch veränderten Proteins, das folgende Schritte umfasst:
a) Immunisieren einer ersten transgenen Maus mit einem Protein von Interesse und Immunisieren einer zweiten transgenen Maus mit einem gentechnisch veränderten Protein, worin das gentechnisch veränderte Protein eine Variante des Proteins von Interesse ist und das Protein von Interesse ein T-Zell-Epitop umfasst, worin sich die Variante von dem Protein von Interesse **dadurch** unterscheidet, dass sie ein verändertes T-Zell-Epitop aufweist;
b) Gewinnen von Serum der ersten und der zweiten immunisierten transgenen Maus;
c) Messen des Serums bezüglich Antigen-spezifischer Immunglobuline; und
d) Vergleichen der immunogenen Reaktion der Variante und des Proteins von Interesse; worin die erste transgene Maus und die zweite transgene Maus HLA DR3/DQ2 sind und worin die Variante und das Protein von Interesse eine unterschiedliche immunogene Reaktion in den transgenen Mäusen bewirken.

2. Verfahren nach Anspruch 1, worin das Protein von Interesse ein Enzym ist.

3. Verfahren nach Anspruch 2, worin das Enzym eine Protease ist.

4. Verfahren nach Anspruch 1, worin das Antigen-spezifische Immunglobulin IgG ist.

5. Verfahren nach Anspruch 1, worin die transgenen HLA-DR3/DQ2-Mäuse mit Mäusen rückgekreuzt wurden, die keine Expression von endogenen I-A-Molekülen der Klasse II zeigen.

6. Verfahren nach Anspruch 1, worin das T-Zell-Epitop mittels Aminosäuresubstitutionen verändert wird.

7. Verfahren nach Anspruch 1, worin das T-Zell-Epitop **dadurch** verändert wird, dass es einen terminalen Abschnitt des das T-Zell-Epitop umfassenden Proteins von Interesse aufweist, der durch einen entsprechenden terminalen Abschnitt eines Homologs des Proteins von Interesse ersetzt ist, worin das Homolog kein T-Zell-Epitop aufweist, das mit dem ersetzten T-Zell-Epitop identisch ist.

8. Verfahren nach Anspruch 1, worin die immunogene Reaktion, die von der Variante bewirkt wird, schwächer ist als die immunogene Reaktion, die von dem Protein von Interesse bewirkt wird.

9. Verfahren nach Anspruch 1, worin die immunogene Antwort, die von der Variante bewirkt wird, stärker ist als die immunogene Antwort, die von dem Protein von Interesse bewirkt wird.

10. Verfahren nach Anspruch 1, worin die immunogene Reaktion eine Voraussage der allergenen Reaktion bei Menschen ermöglicht.

11. Verfahren nach Anspruch 10, worin das Protein von Interesse eine Protease ist.

## Revendications

1. Méthode de détermination du potentiel allergénique d'une protéine manipulée comprenant les étapes de
a) immuniser une première souris transgénique avec une protéine d'intérêt et immuniser une deuxième souris transgénique avec une protéine manipulée, où ladite protéine manipulée est une variante de ladite protéine d'intérêt, et ladite protéine d'intérêt inclut un épitope de cellule-T, où la variante diffère de la protéine d'intérêt en ayant un épitope de cellule T modifié;
b) recueillir le sérum desdites première et deuxième souris transgéniques immunisées;
c) mesurer le sérum en vue des immunoglobulines spécifiques pour l'antigène; et
d) comparer la réponse immunogénique de ladite variante et de ladite protéine d'intérêt; où la première souris transgénique et la deuxième souris transgénique sont HLA DR3/DQ2, et où la variante et la protéine d'intérêt produisent une réponse immunogénique différente dans lesdites souris transgéniques.

2. Méthode selon la revendication 1, dans laquelle ladite protéine d'intérêt est une enzyme.

3. Méthode selon la revendication 2, dans laquelle ladite enzyme est une protéase.

4. Méthode selon la revendication 1, dans laquelle l'immunoglobuline spécifique pour l'antigène est IgG.

5. Méthode selon la revendication 1, dans laquelle les souris transgéniques HLA DR3/DQ2 ont été rétro-croisées avec des souris chez lesquelles l'expression de molécules I-A endogène de classe II est absente.

6. Méthode selon la revendication 1, dans laquelle ledit épitope de cellule-T est modifié avec des substitutions d'acide aminé.

7. Méthode selon la revendication 1, dans laquelle ledit épitope de cellule-T est modifié en ayant une portion terminale de ladite protéine d'intérêt qui inclut ledit épitope de cellule-T remplacé par une portion terminale correspondante d'un homologue de ladite protéine d'intérêt, où ledit homologue ne comprend pas d'épitope de cellule-T identique audit épitope de cellule-T remplacé.

8. Méthode selon la revendication 1, dans laquelle ladite réponse immunogénique produite par la variante est inférieure à la réponse immunogénique produite par la protéine d'intérêt.

9. Méthode selon la revendication 1, dans laquelle ladite réponse immunogénique produite par la variante est supérieure à la réponse immunogénique produite par la protéine d'intérêt.

10. Méthode selon la revendication 1, dans laquelle ladite réponse immunogénique est prédictive de la réponse allergénique chez les humains.

11. Méthode selon la revendication 10, dans laquelle ladite protéine d'intérêt est une protéase.
